(19) Europäisches Patentamt
European Patent Office
Office européen des brevets

(11) **EP 2 492 353 B1**

(12) **EUROPEAN PATENT SPECIFICATION**

(45) Date of publication and mention of the grant of the patent:
**20.08.2014 Bulletin 2014/34**

(51) Int Cl.:
**C12Q 1/68** *(2006.01)*     **C12N 15/09** *(2006.01)*

(21) Application number: **10825084.6**

(22) Date of filing: **25.10.2010**

(86) International application number:
**PCT/JP2010/068869**

(87) International publication number:
**WO 2011/049237 (28.04.2011 Gazette 2011/17)**

(54) **BIOMARKERS FOR PREDICTING THERAPEUTIC EFFECT IN HYPOSENSITIZATION THERAPY**

BIOMARKER ZUR VORHERSAGE DER THERAPEUTISCHEN WIRKUNG EINER HYPOSENSIBILISIERUNGSTHERAPIE

BIOMARQUEURS SERVANT À PRÉDIRE L'EFFET THÉRAPEUTIQUE D'UN TRAITEMENT DE DÉSENSIBILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.10.2009 JP 2009244965**

(43) Date of publication of application:
**29.08.2012 Bulletin 2012/35**

(73) Proprietor: **Tokyo Metropolitan Institute of Medical Science**
**Tokyo 156-8506 (JP)**

(72) Inventors:
• **HIROI, Takachika**
  **Tokyo 156-8506 (JP)**
• **OKUBO, Kimihiro**
  **Tokyo 113-8602 (JP)**

(74) Representative: **Woods, Geoffrey Corlett**
**J A Kemp**
**14 South Square**
**Gray's Inn**
**London WC1R 5JJ (GB)**

(56) References cited:
**WO-A1-2006/024169**

• **LAHDENPERA ET AL: "Kinetics of asthma- and allergy-associated immune response gene expression in peripheral blood mononuclear cells from vaccinated infants after in vitro re-stimulation with vaccine antigen", VACCINE, ELSEVIER LTD, GB, vol. 26, no. 14, 13 February 2008 (2008-02-13), pages 1725-1730, XP022521905, ISSN: 0264-410X, DOI: 10.1016/J.VACCINE.2008.01.041**
• **TAKAO FUJISAWA ET AL: "Biomarkers for Allergen Immunotherapy in Cedar Pollinosis", ALLERGOLOGY INTERNATIONAL, vol. 58, no. 2, 1 January 2009 (2009-01-01), pages 163-170, XP55060755, ISSN: 1323-8930, DOI: 10.2332/allergolint.09-RAI-0097**
• **WALSH K M ET AL: "Association between reduced copy-number at T-cell receptor gamma (TCRgamma) and childhood allergic asthma: A possible role for somatic mosaicism", MUTATION RESEARCH, ELSEVIER, AMSTERDAM, NL, vol. 690, no. 1-2, 7 August 2010 (2010-08-07), pages 89-94, XP027188411, ISSN: 0027-5107 [retrieved on 2010-05-27]**
• **NIEDOSZYTKO M ET AL: "Gene Expression Analysis In Predicting The Effectiveness Of Insect Venom Immunotherapy", JOURNAL OF ALLERGY AND CLINICAL IMMUNOLOGY, ELSEVIER, AMSTERDAM, NL, vol. 125, no. 2, 1 February 2010 (2010-02-01), page AB120, XP026901875, ISSN: 0091-6749, DOI: 10.1016/J.JACI.2009.12.472 [retrieved on 2010-02-01]**

- TAKACHIKA HIROI: 'Kafunsho To Allergy Shikkan ni Taisuru Yobo Oyobi Chiryo ni Kansuru Kenkyu' THE TOKYO METROPOLITAN INSTITUTE OF MEDICAL SCIENCE NENPO HEISEI 21 NENBAN (2009 NEN 9 GATSU) 2009, pages 88 - 100, XP008157806
- NOBUMASA WATANABE ET AL.: 'Allergy Shikkan ni Taisuru Order-made Iryo X. Men'eki Ryoho: Sugi Kafunsho Zekka Men'eki Ryoho no Chiryo Koka ni Kakawaru Biomarker no Kensaku' ALLERGOLOGY & IMMUNOLOGY vol. 17, no. 10, 2010, pages 1730 - 1737, XP008155289
- BUREAU OF SOCIAL WELFARE AND PUBLIC HEALTH, TOKYO METROPOLITAN GOVERNMENT: 'Sugi Kafunsho no Zekka Genkansa Ryoho no Rinsho Kenkyu Kekka' JOURNAL OF THE TOYAKU vol. 32, no. 2, 2010, pages 20 - 21, XP008157994
- BASSETT A.S. ET AL.: 'Copy number variations and risk for schizophrenia in 22qll.2 deletionsyndrome' HUM. MOL. GENET. vol. 17, 2008, pages 4045 - 4053, XP008154717
- HASIN Y. ET AL: 'High-resolution copy-number variation map reflects human olfactoryreceptor diversity and evolution, e1000249' PLOS GENET. vol. 4, no. 11, 2008, pages 1 - 14, XP008154712
- HIROYUKI ABURATANI: 'Copy number variation: clinical significance and analysis methods' SAISHIN IGAKU vol. 64, 2009, pages 821 - 829, XP008154716
- SHUNPEI ISHIKAWA ET AL: 'Hito Genome Kaiseki to Shikkan 2. Hito Genome no Copy Number Variation: CNV' EXPERIMENTAL MEDICINE vol. 25, no. 2, 2007, pages 185 - 191, 138, XP008156981

**Description**

TECHNICAL FIELD

The present invention relates to a biomarker for detecting the effectiveness of allergen immunotherapy upon performing the therapy on a patient with an immediate (Type I) allergy such as a pollen allergy.

BACKGROUND ART

For treating allergy disorders, typically pollen allergies, symptomatic based therapies have been performed in which an anti-allergic drug or the like is administered. Under such circumstances, immunotherapies have been employed for treating the underlying cause (Non-patent document 1). Currently, in Japan, allergen immunotherapy is performed as insured medical care by subcutaneously injecting a causative antigen extract, whereas, in Europe, sublingual allergen immunotherapy has been performed for some years through immunization by putting drops of an antigen extract in a mouth under the tongue (Non-patent documents 2 and 3).
However, the existing allergen immunoterapies require a long-term treatment period of two to three years regardless of the subcutaneously injection or the sublingual method, which, depending on the results of the treatment, may place enormous temporal and financial burdens on the patient.

PRIOR ART DOCUMENTS

Non-Patent Documents

[Non-patent document 1] Rebien W. et al., Eur J Pediatr. 1982 Jul;138(4):341-4.
[Non-patent document 2] Passalacqua G. et al., Canonica GW. BioDrugs. 2001;15(8):509-19. Review.
[Non-patent document 3] Guez S et al., Allergy. 2000 Apr; 55(4):369-75.

SUMMARY OF THE INVENTION

Problems to be Solved by the Invention

In order to reduce the burden including medical expenses placed on the patients, a biomarker that can predict the therapeutic efficacy of allergen immunotherapy needs to be searched. Therefore, the present invention has an objective of providing a biomarker for detecting whether or not allergen immunotherapy is effective when it is performed on a patient suffering from an immediate (Type I) allergy such as a pollen allergy.

MEANS FOR SOLVING THE PROBLEMS

In order to solve the above-described problems, the present inventors have gone through intensive investigation, as a result of which, found that the copy number variations of certain genes are useful for detecting effectiveness of allergen immunotherapy, thereby accomplishing the present invention.
Thus, the present invention is as follows.
A method for detecting effectiveness of allergen immunotherapy for an immediate allergy in a subject, the method comprising the steps of: detecting a copy number variation of at least one gene selected from the following gene group in a specimen to be examined collected from the subject; comparing the obtained result from detecting the copy number variation with data of the copy number variation of the same gene from the parent population to correlate with effectiveness of the allergen immunotherapy:

<Gene group>

AGXT2L2, AZGP1, CAV3, CEP72, CUL4A, CXCR7, C12orf60, C16orf48, C19orf34, DNAJB8, FTH1, GEMIN4, GIGYF2, GJC3, HTT, ITCH, MARK2, METT10D, NAV3, NOB1, PAFAH2, PLEKHG4B, PPFIA1, SCARNA11, SDF2L1, SMPD3, SNORA44, SRP14, ST6GALNAC1, SULF2, TMED6, TYRP1, WFDC13, ZNFX1, NCAM2, PCDH17, CHODL, BTG3, DIAPH3, GBA3, IFRD1, KCNT2, THOC7, TMEM168, BST1, C7orf53, CD38, DCUN1D1, FGFBP1, FOXP2, GLRB, GTF2B, HSP90AB2P, MCCC1, MDFIC, ODF2L, PDGFC, SEP15, SH3GLB1, TDRD3, YEATS2, CCDC127, C14orf180, SIVA1, TNFRSF14, AHNAK2, C14orf79, LOC25845, PLD4, GPR132, LOC389257, BRF1 and ADSSL.

According to the present invention, the result from detecting the copy number variation can be correlated with the effectiveness of the allergen immunotherapy based on any of the following judgment criteria (a) to (d) or a combination thereof:

<Judgment criteria>

(a) the allergen immunotherapy is effective when the copy number of at least one gene selected from the group consisting of NAV3, TYRP1, NCAM2, PCDH17, CHODL, BTG3, DIAPH3, GBA3, IFRD1, KCNT2, THOC7, TMEM168, BST1, C7orf53, CD38, DCUN1D1, FGFBP1, FOXP2, GLRB, GTF2B, HSP90AB2P, MCCC1, MDFIC, ODF2L, PDGFC, SEP15, SH3GLB1, TDRD3 and YEATS2 is smaller than the most frequent copy number (for example, 2) of the same gene from the parent population, preferably 1;

(b) the allergen immunotherapy is effective when the copy number of at least one gene selected from the group consisting of AGXT2L2, AZGP1, CAV3, CEP72, CUL4A, CXCR7, C19orf34, DNAJB8, FTH1, GEMIN4, GIGYF2, GJC3, ITCH, HTT, MARK2, METT10D, PAFAH2, PLEKHG4B, PPFIA1, SCARNA11, SDF2L1, SNORA44, ST6GALNAC1, SULF2, WFDC13, ZNFX1, CCDC127, C14orf180, SIVA1, TNFRSF14, AHNAK2, C14orf79, LOC25845, PLD4, GPR132, LOC389257, BRF1 and ADSSL1 is larger than the most frequent copy number (for example, 2) of the same gene from the parent population, preferably 3;

(c) the allergen immunotherapy is ineffective when the copy number of at least one gene selected from the group consisting of AGXT2L2, CAV3, CEP72, CUL4A, CXCR7, C16orf48, C19orf34, DNAJB8, FTH1, GEMIN4, GIGYF2, GJC3, HTT, ITCH, MARK2, METT10D, NOB1, PAFAH2, PLEKHG4B, SCARNA11, SDF2L1, SMPD3, SNORA44, SRP14, ST6GALNAC1, SULF2, TMED6, WFDC13, ZNFX1, CCDC127, C14orf180, SIVA1, TNFRSF14, AHNAK2, C14orf79, LOC25845, PLD4, GPR132, LOC389257, BRF1 and ADSSL1 is smaller than the most frequent copy number (for example, 2) of the same gene from the parent population, preferably 1; and

(d) the allergen immunotherapy is ineffective when the copy number of at least one gene selected from the group consisting of C12orf60, NAV3, TYRP1, NCAM2, PCDH17, CHODL, BTG3, DIAPH3, GBA3, IFRD1, KCNT2, THOC7, TMEM168, BST1, C7orf53, CD38, FGFBP1, FOXP2, GLRB, GTF2B, HSP90AB2P, MDFIC, ODF2L, PDGFC, SEP15, SH3GLB1 and TDRD3 is larger than the most frequent copy number (for example, 2) of the same gene from the parent population, preferably 3.

A method for detecting effectiveness of allergen immunotherapy for an immediate allergy in a subject, the method comprising the steps of: detecting a copy number variation of at least one gene selected from the following gene group in a specimen to be examined collected from the subject; correlating the obtained result from detecting the copy number variation with effectiveness of the allergen immunotherapy based on any of the following judgment criteria (e) to (h) or a combination thereof:

(e) the allergen immunotherapy is effective when the copy number of at least one gene selected from the group consisting of NAV3, TYRP1, NCAM2, PCDH17, CHODL, BTG3, DIAPH3, GBA3, IFRD1, KCNT2, THOC7, TMEM168, BST1, C7orf53, CD38, DCUN1D1, FGFBP1, FOXP2, GLRB, GTF2B, HSP90AB2P, MCCC1, MDFIC, ODF2L, PDGFC, SEP15, SH3GLB1, TDRD3 and YEATS2 is 1;

(f) the allergen immunotherapy is effective when the copy number of at least one gene selected from the group consisting of AGXT2L2, AZGP1, CAV3, CEP72, CUL4A, CXCR7, C19orf34, DNAJB8, FTH1, GEMIN4, GIGYF2, GJC3, ITCH, HTT, MARK2, METT10D, PAFAH2, PLEKHG4B, PPFIA1, SCARNA11, SDF2L1, SNORA44, ST6GALNAC1, SULF2, WFDC13, ZNFX1, CCDC127, C14orf180, SIVA1, TNFRSF14, AHNAK2, C14orf79, LOC25845, PLD4, GPR132, LOC389257, BRF1 and ADSSL1 is 3;

(g) the allergen immunotherapy is ineffective when the copy number of at least one gene selected from the group consisting of AGXT2L2, CAV3, CEP72, CUL4A, CXCR7, C16orf48, C19orf34, DNAJB8, FTH1, GEMIN4, GIGYF2, GJC3, HTT, ITCH, MARK2, METT10D, NOB1, PAFAH2, PLEKHG4B, SCARNA11, SDF2L1, SMPD3, SNORA44, SRP14, ST6GALNAC1, SULF2, TMED6, WFDC13, ZNFX1, CCDC127, C14orf180, SIVA1, TNFRSF14, AHNAK2, C14orf79, LOC25845, PLD4, GPR132, LOC389257, BRF1 and ADSSL1 is 1; and

(h) the allergen immunotherapy is ineffective when the copy number of at least one gene selected from the group consisting of C12orf60, NAV3, TYRP1, NCAM2, PCDH17, CHODL, BTG3, DIAPH3, GBA3, IFRD1, KCNT2, THOC7, TMEM168, BST1, C7orf53, CD38, FGFBP1, FOXP2, GLRB, GTF2B, HSP90AB2P, MDFIC, ODF2L, PDGFC, SEP15, SH3GLB1 and TDRD3 is 3.

According to the present invention, an example of allergen immunotherapy includes a sublingual allergen immunotherapy. Examples of an immediate allergy include at least one selected from the group consisting of pollen allergy, urticaria, food allergy, mite allergy, allergic rhinitis, bronchial asthma and atopic dermatitis.

EFFECT OF INVENTION

The present invention provides a biomarker that is capable of detecting the efficacy of allergen immunotherapy, when the therapy is performed on a patient currently suffering from an immediate allergy or a subject who may not be currently suffering from an immediate allergy but may in the future. By measuring the copy number variation of the gene collected from the subject, whether or not the allergen immunotherapy is effective at present or in the future can be examined. As a result, a treatment method that is effective for an allergy patient can be provided.

BRIEF DESCRIPTION OF THE DRAWINGS

[Figure 1] A diagram showing a plan for carrying out sublingual allergen immunotherapy.
[Figure 2] Diagrams showing changes in the clinical conditions during sublingual allergen immunotherapy and results from judging the therapeutic efficacy.
[Figure 3] Diagrams showing the relationships between copy number variations and therapeutic efficacy.
[Figure 4] Diagrams showing the relationships between copy number variations and therapeutic efficacy.
[Figure 5] Diagrams showing the relationships between copy number variations and therapeutic efficacy.

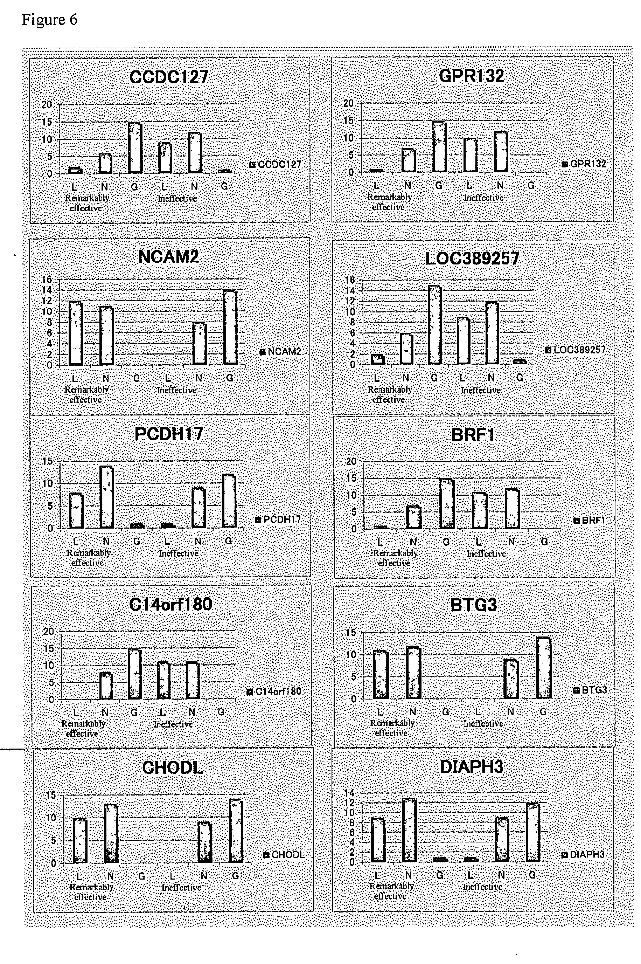

[Figure 6] Diagrams showing the relationships between copy number variations and therapeutic efficacy.

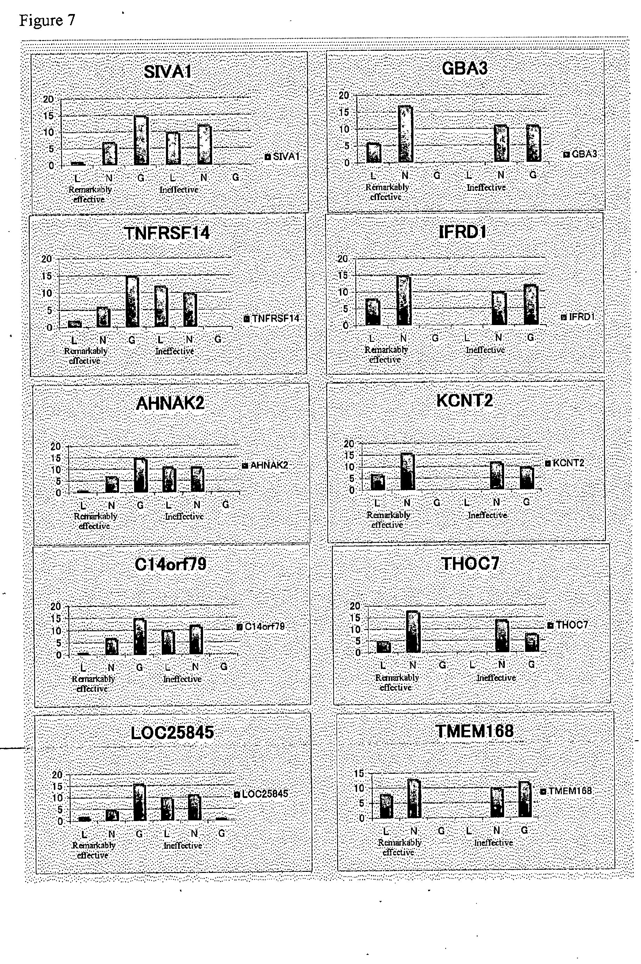

[Figure 7] Diagrams showing the relationships between copy number variations and therapeutic efficacy.

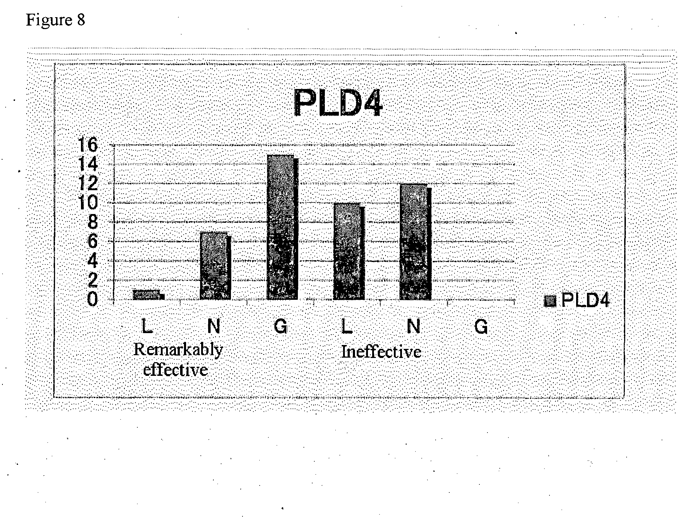

[Figure 8] A diagram showing the relationship between copy number variation and therapeutic efficacy.

EMBODIMENTS FOR CARRYING OUT THE INVENTION

Hereinafter, the present invention will be described in detail.

1. General outline

The present invention relates to a method for detecting the effectiveness of allergen immunotherapy in an immediate allergy patient by using a copy number variation of a gene in a specimen to be examined from a subject (immediate allergy patient, healthy subject, etc.) as an index (correlating with effectiveness of the allergen immunotherapy).
The present inventors have examined the therapeutic efficacy of sublingual allergen immunotherapy in patients with a cedar pollen allergy, and grouped them into a patient group with significant therapeutic efficacy against allergic symptoms (therapeutically effective group) and a patient group without efficacy (therapeutically ineffective group).
For each patient group, genes that correlate with the therapeutic efficacy were detected in a parent population including the therapeutically effective group and the therapeutically ineffective group using a technique called integrated analysis. "Integrated analysis" is a method that collectively puts clinical condition data, results from serological test, mRNA analysis and copy number variation analysis together and comprehensively processes a combination of all or some of these terms by statistical analysis. By doing so, genes that can be employed for detecting effectiveness of allergen immunotherapy, that are, certain genes (listed below) having copy number variations that allow detection of whether the allergen immunotherapy is effective or ineffective.
A copy number variation (CNV) refers to a genomic region whose copy number per cell differs among individual people in a certain population. A human cell generally inherits two (2 copies of) genes from both paternal and maternal sides. However, the number of a certain gene per cell may vary from individual to individual, which may be only one (1 copy) or three (3 copies) or more. Such individual variation in a gene is referred to as CNV For example, the most frequently occurring number of CNV in the above-described parent population is represented as "N (Normal)" (CNV = N), the number of CNV smaller than N is represented as "L (Loss)" (CNV = L), and the number of CNV larger than N is represented as "G (Gain)" (CNV = G).
CNV of a gene contained in a sample of a subject is detected so that whether allergen immunotherapy is effective or ineffective in the subject can be judged prior to the treatment depending on which of the above-described L, N and G the CNV falls into.
Hence, according to the present invention, the copy number variation of a predetermined gene is analyzed so as to correlate the analysis result with efficacy of the allergen immunotherapy to examine whether or not the allergen immunotherapy is effective in the immediate allergy patient.

2. Allergen immunotherapy

Allergen immunotherapy is the only curative treatment among the currently taken allergy treatments, and refers to an immune therapy which disables antigen-specific immune response by gradual exposure to allergens inside the body. Currently, sublingual allergen immunotherapy, subcutaneous allergen immunotherapy and the like are known.

Sublingual allergen immunotherapy is characterized by allowing antigen sensitization to be carried out at home without visiting the specialist and being painless without the need of entering a needle into the body (burden of the patient is small). Sublingual allergen immunotherapy is carried out by putting drops of an antigen extract under the tongue for antigen sensitization, where the antigen sensitization is performed daily starting from lower to higher concentrations in a stepwise manner for the first month of about two years of treatment duration, which is then followed by antigen sensitization twice a week.

On the other hand, subcutaneous allergen immunotherapy is characterized by being painful to some extent for subcutaneously injecting an antigen with a needle, and requires to visit the specialist at least 50 times or more during two years of treatment duration. Similar to sublingual allergen immunotherapy, subcutaneous allergen immunotherapy is carried out by performing antigen sensitization for about two years of treatment duration, where the antigen sensitization is performed daily starting from lower to higher concentrations in a stepwise manner for the first month, which is then followed by antigen sensitization twice a week.

According to the present invention, whether or not the therapy is effective can be detected for either allergen immunotherapy, but it is preferably sublingual allergen immunotherapy which is a allergen immunotherapy recommended by WHO.

3. Subject

A subject as a target of a method of the present invention, namely, a patient suffering from an immediate allergy as a target of allergen immunotherapy for an immediate allergy is not particularly limited as long as the patient presents an immediate (Type I) allergy.

For example, Type I allergies can be classified as follows.

Classification based on antigens: pollen allergy, food allergy and mite allergy.

Classification based on pathological conditions: urticaria, allergic rhinitis, bronchial asthma and atopic dermatitis.

Among them, pollen allergies (cedar pollen allergy, rice pollen allergy, ragweed pollen allergy, Japanese cypress pollen allergy, etc.) are preferable.

Even a healthy subject may have the potential of suffering from an immediate allergy in the future. Therefore, according to the present invention, a healthy subject may also be the subject other than the immediate allergy patients.

4. Analysis of copy number variation

(1) Specimen to be examined

The specimen to be examined may be, for example, a biological specimen from a healthy subject or an immediate allergy patient.

The specimen to be examined used for an analysis of a copy number variation (CNV) may be, for example, blood, mucous membrane of the nose, nasal discharge, sputum or the like collected from the above-described subject, and preferably blood or a component thereof (serum, plasma, etc.). Methods for collecting these biological specimens or the like are known by those skilled in the art.

When the biological specimen to be used for CNV analysis is blood, for example, cells are preferably extracted from a blood specimen to prepare a lysate thereof or to extract RNA (e.g., mRNA) or DNA therefrom. Preparation of a lysate and extraction of DNA may be carried out by using a known method or a commercially available kit. Alternatively, cells extracted from a blood specimen may further be sorted with a Fluorescence Activated Cell Sorter (FACS), MACS (registered trademark) or the like. An example of the extracted cell includes an immune cell. Examples of the immune cell include lymphocytes, macrophages, neutrophils, eosinophils, basophils, monocytes, dendritic cells and plasmacytoid dendritic cells (PDC), where CD4T cells, dendritic cells and basophils are preferable which are suggested to have an association with allergy. Furthermore, the specimen may be messenger RNA (mRNA) extracted from the above-described cell.

When the biological specimen is in a liquid form, it is preferably diluted, for example, in a buffer or the like to be used for the measurement of DNA.

Measurement of CNV is preferably conducted by a microarray method in terms of simple operation.

(2) Genes

According to the present invention, genes used for detecting effectiveness of allergen immunotherapy are as follows.

[Table 1-1]

| Gene | Gene Name | Accession No. |
|---|---|---|
| C12orf60 | chromosome_12_open_reading_frame_60 | NM_175874 |
| SRP14 | signal_recognition‸panticle_14kDa_(homologous_Alu_RNA_binding protein) | NM_003134 |
| C16orf48 | chromosome_16_open_reading_frame_48 | NM_032140 |
| SMPD3 | sphingomyelin_phosphodiesterase_3,_neutral_membrane_ (neutral_sphingomyelinase_II) | NM_018667 |
| TMED6 | transmembrane_emp24_protein_transport_domain_containing_6 | NM_144676 |
| NOB1 | NIN1/RPN12_binding_protein_1_homolog_(S._cerevisiae) | NM_014062 |
| GEMIN4 | gcm_(nuclear_organelle)_associated_protein_4 | NM_015721 |
| SDF2L1 | stromal_cell-derived_factor_2-like_1 | NM_022044 |
| CAV3 | caveolin_3 | NM_001234 |
| DNAJB8 | DnaJ_(Hsp40)_homolog,_subfamily_B,_member_8 | NM_153330 |
| AZGP1 | alpha-2-glycoprotein_1,_zinc-binding | NM_001185 |
| GJC3 | gap_junction_protein,_gamma_3,_30. 2kDa | NM_181538 |
| SNORA44 | small_nucleolar_RNA,_H/ACA_box_44 | NR_002976 |
| PAFAH2 | platelet-activating_factor_acetylhydrolase_2,_40kDa | NM_000437 |
| MARK2 | MAP/microtubule_affinity-regulating_kinase_2 | NM_017490 |
| FTH1 | ferritin,_heavy_polypeptide_1 | NM_002032 |
| PPFIA1 | protein_tyrosine_phosphatase,_receptor_type,_f_polypeptide_ (PTPRF),_interacting_protein_(liprin),_alpha_1 | NM_177423 |
| SCARNA11 | small_Cajal_body-spccifíc_RNA_11 | NR_003012 |

[Table 1-2]

| NAV3 | neuron_navigator_3 | NM_014903 |
|---|---|---|
| CUL4A | cullin_4A | NM_003589 |
| METHOD | methyltransferase_10_domain_containing | NM_024086 |
| ST6GALNAC1 | ST6_(alpha-N-acetyl-neuraminyl-2,3-beta-galactosyl-1,3)-N-acetylgalactosaminide_alpha-2,6-sialyltransferase_1 | NM_018414 |
| C19orf34 | chromosome_19_open_reading_frame_34 | NM_152771 |
| GIGYF2 | GRB10_interacting_GYF_protein_2 | NM_015575 |
| CXCR7 | chemokine_(C-X-C_motif)_receptor_7 | NM_020311 |
| ITCH | itchy_E3_ubiquitin_protein_ligase_homolog_(mouse) | NM_031483 |
| SULF2 | sulfatase_2 | NM_198596 |
| WFDC13 | WAP_four-disulfide_core_domain_13 | NM_172005 |
| ZNFX1 | zinc_finger,_NFX1-type_containing_1 | NM_021035 |
| HTT | huntingtin | NM_002111 |
| CEP72 | centrosomal_protein_72kDa | NM_018140 |

(continued)

| PLEKHG4B | pleckstrin_homology_domain_containing_family_G_ (with_RhoGof_domain)_membor_4B | NM_052909 |
|---|---|---|
| AGXT2L2 | alanine-glyoxylate_aminotransferase_2-like_2 | NM_153373 |
| TYRP1 | tyrosinase-related_protein_1 | NM_000550 |
| CCDC127 | coiled-coil domain containing 127 | NM_145265 |
| NCAM2 | neural cell adhesion molecule 2 | NM_001113208 |
| PCDH17 | protocadherin 17 | NM_001040429 |
| C14orf180 | chromosome 14 open reading frame 180 | NM_001008404 |
| CHODL | chondrolectin | NM_139134 |
| SIVA1 | apoptosis-inducing factor | NM_001045673 |
| TNFRSF14 | tumor necrosis factor receptor superfamily, member 14 (herpesvirus entry mediator) | NM_003820 |
| AHNAK2 | nucleoprotein 2 | NM_138420 |
| C14orf79 | chromosome 14 open reading frame 79 | |
| LOC25845 | | NR_024158. |
| PLD4 | phospholipase D family, member 4 | NM_178911 |
| GPR132 | G protein-coupled receptor 132 | NM_013345 |
| L0C389257 | leucine rich repeat containing 14B | NM_001080478 |
| BRF1 | BRF1 homolog, subunit of RNA polymerase III transcription initiation factor IIIB (S. cerevisiae) | XM_421405 |
| BTG3 | BTG family, member 3 | NM_019290 |
| DIAPH3 | diaphanous homolog 3 (Drosophila) | XM_224392 |
| GBA3 | glucosidase, beta, acid 3 (cytosolic) | NM_001128432 |
| IFRD1 | interferon-related developmental regulator 1 | NM_001007245 |

[Table 1-3]

| KCNT2 | potassium channel, subfamily T, member 2 | NM_198503 |
|---|---|---|
| TH0C7 | TH0 complex 7 homolog (Drosophila) | NM_025075 |
| TMEM168 | transmembrane protein 168 | NM_022484 |
| ADSSL1 | adenylosuccinate synthase like 1 | NM_152328 |
| BST1 | bone marrow stromal cell antigen 1 | NM_004334 |
| C7orf53 | chromosome 7 open reading frame 53 | NM_001134468 |
| CD38 | CD38 molecule | NM_001775 |
| DCUN1D1 | DCN1, defective in cullin neddylation 1, domain containing 1 | NM_020640 |
| FGFBP1 | fibroblast growth factor binding protein 1 | NM_005130 |
| FOXP2 | forkhead box P2 | NM_001172766 |
| GLRB | glycine receptor, beta | NM_000824 |
| GTF2B | general transcription factor IIB | NM_001514 |
| HSP90AB2P | heat shock protein 90kDa alpha (cytosolic), class B member 2 (pseudogene) | NR_003132 |

(continued)

| MCCC1 | methylcrotonoyl-CoA carboxylase 1 (alpha) | NM_020166 |
|---|---|---|
| MDFIC | MyoD family inhibitor domain containing | NM_001166345 |
| 0DF2L | outer dense fiber of sperm tails 2-like | NM_001007022 |
| PDGFC | platelet derived growth factor C | NM_016205 |
| SEP15 | 15 kDa selenoprotein | NM_004261 |
| SH3GLB1 | SH3-domain GRB2-like endophilin B1 | NM_016009 |
| TDRD3 | tudor domain containing 3 | NM_001146070 |
| YEATS2 | YEATS domain containing 2 | NM_018023 |

The above-listed genes were extracted by performing an integrated analysis (see Examples).

According to the present invention, at least one gene among the above-mentioned genes can be used.

Moreover, nucleotide sequence information of the genes are easily accessible from Accession numbers indicated above.

(3) Technique of analyzing gene copy variation

Examples of a method for analyzing a CNV variation of a gene according to the present invention include known methods such as a microarray method and a sequencing method, and, for example, may also use a commercially available kit (for example, "Agilent SurePrint G3 Human CNV microarray kit 2 x 400K" (Agilent Technologies)).

A DNA microarray has one ends of nucleotide probes fixed on a support in an array, and includes a DNA chip, a Gene chip, a microchip, a bead array and the like. An example of a DNA microarray assay such as a DNA chip includes GeneChip assay (Affymetrix). GeneChip technique utilizes a small-scale high-density microarray of oligonucleotide probes attached to a chip.

A sequencing method is a method of analyzing the presence or the absence of a copy number variation by amplifying a region including the copy number variation by PCR and sequencing the DNA sequence with a Dye Terminator or the like (Sambrook, Fritsch and Maniatis, "Molecular Cloning: A Laboratory Manual" 2nd Edition (1989), Cold Spring Harbor Laboratory Press).

According to a detection method like the one exemplified above, an oligonucleotide prepared to include a gene of interest is used as a probe or a primer. Accordingly, the present invention also provides an oligonucleotide prepared to include each of the genes of interest.

The oligonucleotide primer or oligonucleotide probe designed as described above may be chemically synthesized by a known technique or method but generally they are synthesized using a commercially available chemical synthesizer.

Moreover, the operation can be automated by adding a fluorescent label (e.g., FITC, FAM, VIC, Redmond Dye or the like) and a quencher for the fluorescent label to the probe.

(4) Judgment criteria

The detection results may be correlated with effectiveness of the allergen immunotherapy based on any of the following judgment criteria (a) to (d) or a combination thereof:

(a) the allergen immunotherapy is effective when the copy number of at least one gene selected from the group consisting of NAV3, TYRP1, NCAM2, PCDH17, CHODL, BTG3, DIAPH3, GBA3, IFRD1, KCNT2, THOC7, TMEM168, BST1, C7orf53, CD38, DCUN1D1, FGFBP1, FOXP2, GLRB, GTF2B, HSP90AB2P, MCCC1, MDFIC, ODF2L, PDGFC, SEP15, SH3GLB1, TDRD3 and YEATS2 is smaller than the most frequent copy number (for example, 2) of the same gene from the parent population, preferably 1;

(b) the allergen immunotherapy is effective when the copy number of at least one gene selected from the group consisting of AGXT2L2, AZGP1, CAV3, CEP72, CUL4A, CXCR7, C19orf34, DNAJB8, FTH1, GEMIN4, GIGYF2, GJC3, ITCH, HTT, MARK2, METT10D, PAFAH2, PLEKHG4B, PPFIA1, SCARNA11, SDF2L1, SNORA44, ST6GALNAC1, SULF2, WFDC13, ZNFX1, CCDC127, C14orf180, SIVA1, TNFRSF14, AHNAK2, C14orf79, LOC25845, PLD4, GPR132, LOC389257, BRF1 and ADSSL1 is larger than the most frequent copy number (for example, 2) of the same gene from the parent population, preferably 3;

(c) the allergen immunotherapy is ineffective when the copy number of at least one gene selected from the group consisting of AGXT2L2, CAV3, CEP72, CUL4A, CXCR7, C16orf48, C19orf34, DNAJB8, FTH1, GEMIN4, GIGYF2, GJC3, HTT, ITCH, MARK2, METT10D, NOB1, PAFAH2, PLEKHG4B, SCARNA11, SDF2L1, SMPD3, SNORA44, SRP14, ST6GALNAC1, SULF2, TMED6, WFDC13, ZNFX1, CCDC127, C14orf180, SIVA1, TNFRSF14, AHNAK2, C14orf79, LOC25845, PLD4, GPR132, LOC389257, BRF1 and ADSSL1 is smaller than the most frequent copy number (for example, 2) of the same gene from the parent population, preferably 1; and

(d) the allergen immunotherapy is ineffective when the copy number of at least one gene selected from the group consisting of C12orf60, NAV3, TYRP1, NCAM2, PCDH17, CHODL, BTG3, DIAPH3, GBA3, IFRD1, KCNT2, THOC7, TMEM168, BST1, C7orf53, CD38, FGFBP1, FOXP2, GLRB, GTF2B, HSP90AB2P, MDFIC, ODF2L, PDGFC, SEP15, SH3GLB1 and TDRD3 is larger than the most frequent copy number (for example, 2) of the same gene from the parent population, preferably 3.

Herein, the terms "remarkably effective" and "effective" are synonymous.

Those skilled in the art can appropriately determine which of the judgment criteria (a) to (d) should a target gene used with the present invention be sorted into upon correlating the copy number of the gene with effectiveness of the allergen immunotherapy. For example, the target genes from the parent population of the patients undergoing hyposensitization treatment or a subpopulation extracted from the parent population are classified into four groups based on their copy numbers (whether CNV is L or G) and the therapeutic efficacy (remarkably effective or ineffective). Then, for the groups with CNV of L or G, difference between the number of people belonging to the remarkably effective group and the number of people belonging to the ineffective group is calculated. A large difference means that judgment can be made as to which CNV gives remarkable effectiveness or ineffectiveness in the therapeutic efficacy.

For example, in Example 1, looking at CNV of GEMIN4 gene (Table 9) from 25 patients extracted from the parent population of the treated patients, 8 patients whose therapeutic efficacy was ineffective had CNV of 1 while the number of patients whose therapeutic efficacy was remarkably effective with CNV of 1 was zero (0). On the other hand, 12 patients whose therapeutic efficacy was remarkably effective had CNV of 3 while the number of patients whose therapeutic efficacy was ineffective with CNV of 3 was only one. The difference between the number of remarkably effective patients with CNV of 1 and the number of ineffective patients with CNV of 1 is 0 - 8 = -8. In addition, the difference between the number of remarkably effective patient with CNV of 3 and the number of ineffective patient with CNV of 1 is 12 - 1 = 11. When the absolute value of this difference makes up at least 8% or more, preferably 12% or more and more preferably 25% or more of the patients subjected to the calculation, the gene can be included in the target genes that can be used for judging remarkable effectiveness or ineffectiveness. In the later-described example, since 24 people from the remarkably effective group and 25 people from the ineffective group were analyzed, a gene can be used as a target gene for judging remarkable effectiveness or ineffectiveness when the difference in the numbers of the patients is 8% or more of the total number of the patients, that is, two or more people (24 x 0.08 ≈ 2 or 25 x 0.08 = 2). The judgment can be "remarkably effective" when the result obtained by subtracting the number of ineffective patients from the number of remarkably effective patients is positive, and "ineffective" when negative. In the case where the number of the remarkably effective patients is subtracted from the number of the ineffective patients, the judgment results based on positive or negative value would be the other way around.

The present invention is also capable of calculating the difference between the number of the effective patients and the number of the ineffective patients in a more strict way. Although the calculation of the difference was conducted without considering the patients with CNV of N in the above-described example, the calculation can also take place in consideration of the patients with CNV of N. However, since the number of the patients with CNV ofN does not serve as a criterion for sorting genes into the judgment criteria, it is considered as a background value and thus, preferably, a predetermined proportion, for example, 50% or more, 60% or more, 70% or more, 80% or more, 90% or more or whole (100%) number of patients in the parent population or the subpopulation is subtracted from the parent population. In this case, when the number of patients resulting from subtraction from the patients belonging to CNV = N is negative, the CNV = N patient is calculated as 0 (zero). This value is used as a correction value ("CNV = N correction value"), for example, in the following calculation.

The rates of remarkable effectiveness are calculated by the following calculation formula where CNV of L and G are represented as "CNV = L" and "CNV = G", respectively.

(1)

Rate of remarkable effectiveness with CNV = L

[{Number of remarkably effective cases with CNV = L/(Number of people with CNV = L + CNV = N Correction value + Number of people with CNV = G, in the remarkably effective groups)} - {Number of ineffective cases with CNV = L/(Number of people with CNV = L + CNV = N Correction value + Number of people with CNV = G, in the ineffective groups)}] x 100

(2)

Rate of remarkable efficacy with CNV = G

[{Number of remarkably effective cases with CNV = G/(Number of people with CNV = L + CNV = N Correction value + Number of people with CNV = G, in the remarkably effective groups)} - {Number of ineffective cases with CNV = G/(Number of people with CNV = L + CNV = N Correction value + Number of people with CNV = G, in the ineffective groups)}] x 100

(3) When the value obtained in (1) or (2) above is equal to or higher than the criterion value (positive number), the gene targeted by the calculation is selected as a marker gene for remarkable effectiveness, whereas when the value is equal to or lower than the criterion value (negative number), the gene targeted by the calculation is selected as a marker gene for ineffectiveness. According to the above-described calculation formula, the target gene can be judged as a gene that indicates remarkable effectiveness when the resulting value is positive whereas the target gene can be judged as a gene that indicates ineffectiveness when the resulting value is negative.

For example, when the rates of remarkable effectiveness are calculated with respect to the above-mentioned GEMIN4 gene under the above-described conditions, the rates of remarkable effectiveness will be as follows:

Formula (1): (0/12 - 8/9) x 100 = -88.9

Formula (2): (12/12 - 1/9) x 100 = 88.9

Here, CNV = 2 (CNV = N) is considered as background, where a portion of the number of people corresponding to 80% of the parent population (for example, 24 (CNV=1) x 0.8 = 19 people) was subtracted. Since subtraction of 19 people gave a negative number, it was considered as 0.

Then, if the absolute value of the criterion targeted for selection is set to 50, the gene is judged to indicate remarkable effectiveness when the rate is +50 or higher and the gene is judged to indicate ineffectiveness when the rate is -50. In the case of the above-described example, the selection criterion of the rate of remarkable effectiveness was set to 50 (absolute value) but it may be set to 10, 20, 30, 40, 50, 60, 70, 80 or 90 according to the present invention depending on the purpose of the analysis.

Accordingly, since the value obtained in Formula (1) is a negative value, GEMIN4 gene with CNV of 1 is selected as a gene that indicates that the allergen immunotherapy is ineffective, whereas GEMIN4 gene with CNV of 3 is selected as a gene that indicates that the allergen immunotherapy is remarkably effective.

Furthermore, according to the present invention, in order to select a gene for judging between remarkable effectiveness and effectiveness, various statistical analyzing techniques such as multivariate analysis may be used. Examples of a multivariate analysis method include a multiple regression analysis, a discriminant analysis, a principal component analysis, a factor analysis and the like. See Example 2 described below for details.

On the other hand, according to the method of the present invention, the results from the CNV measurement of the above-described gene obtained from a predetermined number of patients (primary parent population) can be used as reference data so that the effectiveness of allergen immunotherapy can be judged for a subject outside the primary parent population by comparing the CNV of each gene of the subject with the reference data.

The data of the subject outside the primary parent population may also be integrated into the values of the primary parent population, and the expression level thereof may again be subjected to data processing (averaging, etc.) so as to increase the number of the target subjects (the parent population). By increasing the number of cases, accuracy of the CNV of each gene and thus accuracy of detection or diagnosis can be improved.

In an actual clinical setting, the method of the present invention can be applied to samples individually collected from patients, subjects who had medical checkup or the like so as to judge whether the allergen immunotherapy is remarkably effective or ineffective to the patients or the subjects before starting the actual treatment. Specifically, genomic DNA is collected from the sample to detect the copy number of the gene associated with the remarkable effectiveness or ineffectiveness of the allergen immunotherapy. When the copy number is L or G, the patient or the subject can be judged to show remarkable effectiveness or ineffectiveness to allergen immunotherapy.

For example, in the case where CNV of NAV3 gene present in genomic DNA collected from a certain patient is L, this patient can be expected to show remarkable effectiveness to allergen immunotherapy.

Moreover, copy numbers were examined for M number of genes among NAV3, TYRP1, NCAM2, PCDH17, CHODL, BTG3, DIAPH3, GBA3, IFRD1, KCNT2, THOC7, TMEM168, BST1, C7orf53, CD38, DCUN1D1, FGFBP1, FOXP2, GLRB, GTF2B, HSP90AB2P, MCCC1, MDFIC, ODF2L, PDGFC, SEP15, SH3GLB1, TDRD3 and YEATS2 genes in a genome collected from a certain patient. If the copy number is L for some of the genes examined but N or G for the rest of the genes, the copy numbers of L, N and G are scored as +1, 0 and -1, respectively, and the scores are summed up for each gene. From the resulting sum, judgment of remarkable effectiveness or ineffectiveness can comprehensively be made. In this case, when the sum is 50% or more, preferably 60% or more, 70% or more, 80% or more, 90% or more or 100% with respect to M, the patient can be judged to show remarkable effectiveness to allergen immunotherapy.

Alternatively, the sum of the scores assigned to the copy numbers of a certain gene or a plurality of genes can be presented to the patient while explaining the correlation between the copy number or the sum with remarkable effectiveness or ineffectiveness of the allergen immunotherapy so that it can be used as a risk factor for the patient himself/herself to judge whether to actually perform or continue the allergen immunotherapy with the copy number or the sum in mind.

Furthermore, the detection result may be used, for example, as the main information or subsidiary information for making a confirmation diagnosis of the therapeutic efficacy against an immediate allergy. In order to make a diagnosis for confirming that the immediate allergy has been treated, judgment can be made comprehensively by combining the above-described detection results with at least one selected from the results of physical remarks, the results from a serological examination and the like after the allergen immunotherapy.

## 5. Microarray and Kit

Furthermore, one ends of the oligonucleotides of the present invention can be fixed to a support such as glass, silicon or gel to prepare a microarray. The oligonucleotide array can be produced, for example, by light irradiation chemical synthesis method (Affymetrix) in which a solid-phase chemical synthesis method is combined with a photolithography production technique employed in the semiconductor industry. In order to clearly define the border between the chemical reaction sites on the chip, a particular chemical synthesis process is carried out using a photolithography mask, thereby constructing a high-density array having oligonucleotide probes attached to predetermined positions of the array.

Moreover, according to another aspect of the present invention, a kit for detecting CNV variations of a gene, which includes oligonucleotides of the present invention and/or a microarray prepared with the oligonucleotides is provided. Such a kit may also include, other than the oligonucleotides of the present invention and/or the microarray prepared with the oligonucleotides, a solution for detection reaction, an oligonucleotide as a control, a vessel used for detection reaction, instructions and the like.

Hereinafter, the present invention will be described more specifically by means of examples, although these examples are provided for the purpose of illustrating the present invention and do not limit the present invention.

Example 1

<Method >

1. Sorting between therapeutically effective group and ineffective group

First, therapeutic efficacy of sublingual allergen immunotherapy for cedar pollen allergy was examined during two years of administration of a pollen allergy therapeutic drug (a allergen immunotherapy drug). The allergen immunotherapy drug (standardized cedar pollen extract (Torii Pharmaceutical Co., Ltd.)) was given under the administration schedule shown in Table 2.

[Table 2]

|  | Week 1 2 JAU/ml | Week 2 20 JAU/ml | Week 3 200 JAU/ml | Week 4 2000 JAU/ml | Week 5 and later 2000 JAU/ml |
|---|---|---|---|---|---|
| Day 1 | drop | drop | drop | drop | |
| Day 2 | 2 drops | 2 drops | 2 drops | 2 drops | |
| Day 3 | 3 drops | drops | 3 drops | 4 drops | 20 drops |
| Day 4 | 4 drops | 4drops | 4 drops | 8 drops | |
| Day 5 | 6 drops | 6 drops | 6 drops | 12 drops | |
| Day 6 | 8 drops | 8 drops | 8 drops | 18 drops | |
| Day 7 | 10 drops | 10 drops | 10 drops | 20 drops | 20 drops |

Patients were classified into a group with remarkable therapeutic efficacy (therapeutically effective group) and a group with no therapeutic efficacy (group in which conditions worsened or did not change with the treatment: therapeutically ineffective group) with respect to the allergic symptoms. In doing so, conditions prior to treatment in August, *Heisei* 18 (2006), conditions in the middle of the treatment in April, *Heisei* 19
(2007) and conditions in the later period of the treatment in April *Heisei* 20 (2008) were sorted into the effective group and the ineffective group of the pollen allergy treatment using, as indicators, clinical conditions obtained by comprehensively scoring the conditions of the mucous membrane of the nose in the allergy diary.

2. Collection of serum

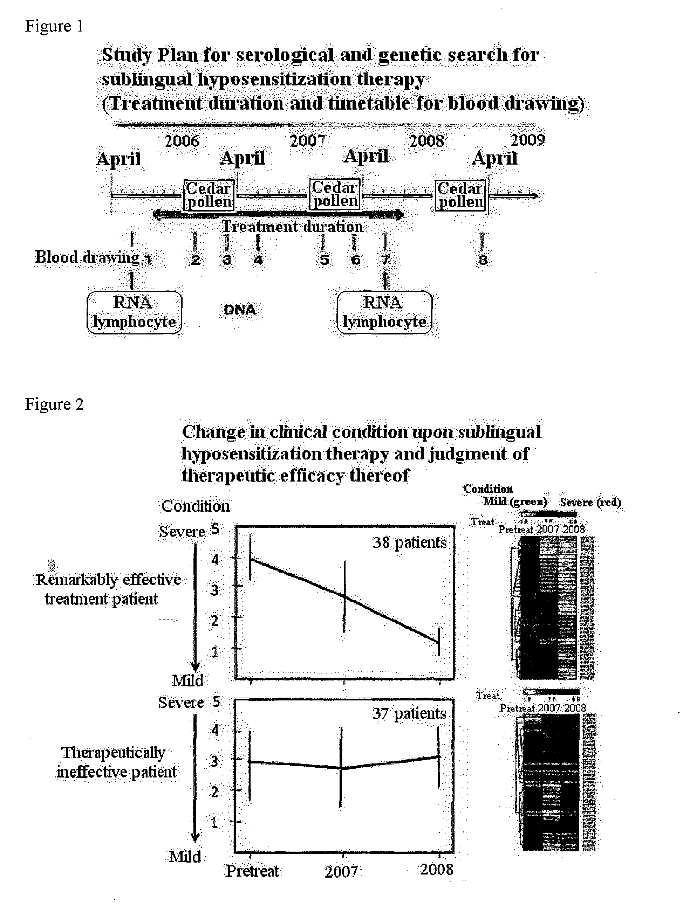

During the period of the sublingual allergen immunotherapy, bloods were drawn 8 times under the schedule shown in Figure 1 (blood drawings 1 to 8). The amount of blood per single blood drawing was 8 ml per person, which was subjected to centrifugal operation immediately after the blood drawing to isolate the serum. The isolated serum was dispensed 1 ml each and stored at -80°C until use.

3. Collection of DNA and RNA

RNA samples before and after the sublingual allergen immunotherapy were collected, separately from the above-described serum, upon blood drawing 1 (prior to treatment) and blood drawing 7 (following treatment) shown in Figure 1.
Sublingual allergen immunotherapy was carried out by administering actual drugs to 202 patients for two year from August, *Heisei* 18 (2006). Therapy was carried out in eight hospitals in Japan. Bloods were sampled under the schedule of blood drawings 1 to 8 shown in Figure 1, and DNA samples were extracted upon blood drawing 2 shown in Figure 1.
The amount of blood drawn per single blood drawing was 8 ml per person, which was subjected to cell isolation operation immediately after the blood drawing with a flow cytometry (Becton, FACSaria) to isolate into CD4T cell, a dendritic cell and the like. The isolated cells were each suspended in a cell freeze preservation medium and then stored at -80°C until use.

Along with the therapeutic efficacy judgment in June, *Heisei* 20 (2008), 25 patients were sorted out from each of the therapeutically effective and ineffective samples that had been classified, and their RNAs were purified according to an ordinary technique. The purified messenger RNA (mRNA) was immediately subjected to a gene analyzer (DNA chip from Affymetrix).

Meanwhile, DNA was collected once during the examination period as described above, for which the amount of blood drawn was 8 ml per person. Centrifugal operation was performed immediately after the blood drawing to isolate blood cells. DNA was collected from the resulting blood cells according to an ordinary technique. The purified and isolated DNA was stored at -80°C until gene analysis.

4. Serological test

For the sera obtained above, the cedar pollen-specific IgE level (RISA-cedar) and the total IgE level (RAST) were measured by ELISA according to an ordinary technique. Fifty types of immune-related humoral factors in the blood (Table 3) were measured according to a fluorescent bead measurement method (Bio-plex from Bio-Rad).

[Table 3]

**Measurement terms for immune-related humoral factors**

**Human Group I** — **27-Plex Panel (171-A11127)**

| | | | | | | | | |
|---|---|---|---|---|---|---|---|---|
| Cytokine group | IL-1$\beta$ | IL-1R$\alpha$ | IL-2 | IL-4 | IL-5 | IL-6 | IL-7 | IL-9 |
| | IL-10 | IL-12 | IL-13 | IL-15 | IL-17 | IFN-$\gamma$ | TNF-$\alpha$ | |
| Chemokine group | IL-8 | Eotaxin | IP-10 | MCP-1(MCAF) | MIP-1$\alpha$ | MIP-1$\beta$ | RANTE S | |
| Growth factor group | Basic FGF | G-CSF | GM-CSF | PDGF-BB | VEGF | | | |

**Human Group II** — **23-Plex panel (171-A11123)**

| | | | | | | | | | |
|---|---|---|---|---|---|---|---|---|---|
| Cytokine group | IL-1$\alpha$ | IL-2R$\alpha$ | IL-3 | IL-12 (p40) | IL-16 | IL-18 | IFN-$\alpha$2 | TNF-$\beta$ | TRAIL |
| Chemokine group | CTACK | GRO-$\alpha$ | ICAM-1 | MCP-3 | MIF | MIG | VCAM-1 | | |
| Growth factor group | HGF | LIF | M-CSF | $\beta$-NGF | SCF | SCGF-$\beta$ | SDF1$\alpha$ | | |

5. Integrated analysis

Clinical study was conducted by actual drug administration in a scale of about 200 patients. The treatment duration was about two years, and data such as clinical conditions were obtained for about 150 patients at the end. The conditions (severity levels) were scored to classify the therapeutic efficacy in detail. As a result, classification gave five groups, among which the group with the highest therapeutic efficacy (remarkably effective treatment group) and the worst group (therapeutically ineffective group) were extracted. Biomaterials of the top 24 patients and the bottom 25 patients from these groups were subjected to the following research analysis.

Hereinafter, the detail will be described.

Sublingual allergen immunotherapy was conducted from the beginning of the treatment for two years and 154 patients who were interviewed about their clinical conditions in June, 2008 (*Heisei* 20) were subjected to analysis. In addition to the previous assessment of the conditions, data of QOL and the like were added for further research and obtained the results shown in Figure 2.
In Figure 2, as shown in the upper left graph, the average severity level of the condition shows that the conditions were alleviated year by year as compared to the result from the pre-study checkup and that the treatment succeeded. 38 people were extracted from the remarkably effective treatment group. On the other hand, the lower graph shows no change or rather an increase in the average severity level of the allergic symptoms each year with respect to the pre-study checkup. This indicates a patient group to whom two years of sublingual allergen immunotherapy turned out to be not effective. 37 people were extracted from this therapeutically ineffective group. Furthermore, the patients extracted from each group are vertically arranged in order starting from the patient with the worst therapeutic efficacy with respect to the percentile shown by colors in the right panels in Figure 2.
The conditions (severe conditions) with the highest severity level of 5 are shown in red while the lowest conditions (mild conditions) of 1 are shown in green. Conditions that lie between the highest and the lowest severity levels are shown in a gradient shading from red to green. Patients extracted by therapeutic efficacy are vertically arranged in order starting from the highest efficacy for the remarkably effective group and from the lowest efficacy for the therapeutically ineffective group. The top 24 and 25 patients from the remarkably effective group and the therapeutically ineffective group, respectively, were subjected to the subsequent analysis.

<Analysis terms and integrated analysis method>

(1) Analysis of clinical conditions

The severity levels at the beginning of the allergen immunotherapy (as of 2006) were organized into 5 stages from 1 (mild) to 5 (severe) to be used as indicators of severity. As indicators of alleviation upon the efficacy judgment after two years of allergen immunotherapy (as of 2008), elimination of the symptom or improvement in the severity level by two stages was defined as grade 1 (remarkably effective treatment group), improvement in the severity level by one stage as grade 2, and no change or worsening as grade 3 (therapeutically ineffective group).

(2) Serological test

In addition to the above-described 50 immunological humoral factors (Table 3), the antigen-specific IgE level and the total IgE level were added.

(3) mRNA analysis

mRNA derived from CD4 + T cells fractionated by FACS from the blood samples drawn at blood drawing points 1 and 7, i.e., upon assessment of severity or alleviation was used for detection with GeneChip (R) Human Gene 1.9 ST array from Affymetrix. As a pre-process, normalization of mRNA expression level was carried out using RMA algorithm (1) with aroma.affymetrix package [R program] to obtain corrected gene mRNA value.

(4) CNV analysis

Agilent Sure Print 3G CNV microarray kit was used to detect total of 49 patients from the patient groups (24 from the remarkably effective treatment group and 25 from the therapeutically ineffective group) to detect the CNV copy numbers according to the following method.
For genomic DNA extracted from peripheral blood-derived blood samples, Log2 value resulting from competitive hybridization with HapMap Japanese sample NA19000 reference DNA as a pre-process was normalized with Normtools (2) based on the population data. The corrected Log2 values were used as Copy Number Aberration (CNA) for segment judgment by CBS algorithm [R program], thereby obtaining CNV data. Based on this CNV data, CNV copy number was judged by gene unit using [median population +/-2SD population*median individual] of the CNV data value as a threshold (Copy number = 2).

(5) Analysis models and integrated analysis

1) Severity of pollen allergy in 2006, CNV: mRNA (blood drawing point 1 prior to treatment)
2) Efficacy assessment (alleviation) in 2008, CNV: mRNA (blood drawing point 7 after treatment)

Analysis model 1) was analyzed for relationship with severity reflecting the conditions of pollen allergy in an individual prior to the commencement of the clinical study in 2006, while analysis model 2) was analyzed for relationship with alleviation reflecting the judgment of efficacy of allergen immunotherapy by the clinical study in 2008.

For 18,945 genes intertwined with gene symbols based on mRNA data and CNV data, a linear model regression analysis was conducted using three parameters, i.e., serological test terms, mRNA values and CNV values of the above-mentioned analysis models 1) and 2) as explanatory variables and severity level of pollen allergy in the spring of 2006 and the value of efficacy judgment of the cedar pollen extract trial in the spring of 2008 as dependent variables.

P values and contribution levels (corrected $R^2$ values) were calculated for about 3.63 million analyses as analysis models 1) and 2). Sorting was conducted for analysis models 1) and 2) by filtering using contribution levels of the analysis models to the regression line as criteria. Thereafter, relationship with P value was assessed as Bonferroni corrected P value. The resulting analysis models and each explanatory variable were examined in detail in terms of the association between the clinical serum marker value, the mRNA value or the CNV value and the clinical phenotype, thereby obtaining final candidate models.

Specifically, with respect to each grade of analysis model 1) (stages 1 to 5) and analysis model 2) (stages 1 to 3), R-2.9 program was used to calculate $R^2$ value as the contribution level and P value, for a combination in which any one term is modified in proportion to the measure of other term in a non-additive association, as an interaction term of three parameter terms, i.e., (a) mRNA gene Log2 strength, (b) relative Log2 ratio of the CNV segment on the gene and (c) serological test terms determined before and after the mRNA collection/blood drawing points. Here, as to the serological test term, values from blood drawings 1 and 2 and blood drawings 5 and 6 were made to correspond to analysis models 1) and 2), respectively. After the calculation of the statistic values, first-stage filtering was performed on each analysis model to extract combinations as candidate combinations of $R^2 > 0.2$ and P value $< 0.05$. As a result, 79 and 396 combinations were obtained for analysis models 1) and 2), respectively. Among these combinations, combinations were obtained by second-stage filtering in which the results of copy number judgment obtained in (4) CNV analysis above were once again assessed with respect to the CNV copy number of the gene.

As a result, 20 and 227 combinations were obtained in analysis models 1) and 2), respectively while 12 and 86 genes were obtained in analysis models 1) and 2), respectively. Among these genes, general linear regression model analysis was performed using analysis model 2) and serological test term as the third-stage filtering so as to extract significant serological test term (P value $< 0.05$) from this model analysis. As a result, 22 genes with 37 combinations were obtained.

<CNV analysis and results from integrated analysis>

From the above-described results, 12 genes (Table 4) were obtained for analysis model 1) up to the second-stage filtering, whereas 22 genes were obtained for analysis model 2) up to the third-stage filtering (Table 5), resulting in total of 34 genes from analysis models 1) and 2) (Table 6).

[Table 4]

| Gene | Gene name | Serum marker | Chromosome | Chromosomal band | N | P-value | R_squre |
|---|---|---|---|---|---|---|---|
| C12orf60 | chromosome_12_open_reading_frame_60 | IL,1B_1 MIP.1B_1 | 12 | 12p12.3 | 42 | 0.000943851 | 0.213265968 |
| SRP14 | signal_recognition_particle_14kDa_(hotnologous _Alu_RNA_binding_protein) | MCP.3_1 | 15 | 15q22 | 41 | 0.001224931 | 0.204076074 |
| C16orf48 | chromosome_16_open_reading frame 48 | _CTACK_1_ MIF_1 | 16 | 16q22.1 | 41 | 0.001187278 | 0.209682617 |
| SMPD3 | sphingomyelin_phosphodiesterase_3,_neutral_ membrane_(neukral sphingomyelinase_ II) | IL.12p40_1 RAST_1 | 16 | 16q22.1 | 41 / 48 | 0.001408257 / 0.000838055 | 0.203513736 / 0.222160983 |
| TMED6 | transmembrane_emp24_protein transport_dom ain_containing_6 | IL.12p40_1 | 16 | 16q22.1 | 41 | 0.001433575 | 0.202867716 |
| NOB1 | NIN1/RPN12_binding_protein_1_homolog_(s._c erevisiae) | CTACK_1 | 16 | 16q22.3 | 41 | 0.000420248 | 0.214315316 |
| GEMIN4 | gem_(nuclear_organelle)_associated_protein_4 | PDGF.BB_2 | 17 | 17p13 | 48 | 0.001330579 | 0.205568236 |
| SDF2L1 | stromal_cell-derived_factor_2-like_1 | PDGF.BB_2 | 22 | 22q11.21 | 48 | 0.001358123 | 0.204826754 |
|  |  | SCF_1 |  |  | 41 | 0.000622967 | 0.202088296 |
| CAV3 | caveolin_3 | MIP.1A_1 MIP.1B_1 | 3 | 3p25 | 42 | 0.00061778 | 0.202349482 |
| DNAJB8 | DnaJ_(Hsp40)_homolog,_subfamily_B,_membe r_8 | TNF.B_1 TNF.B_2 | 3 | 3q21.3 | 41 / 48 | 0.001539543 / 0.001275146 | 0.200273444 / 0.202652638 |
| AZGP1 | alpha-2-glycoprotein_1,_zinc-binding | PDGF.BB 2 | 7 | 7q22.1 | 48 | 0.000969229 | 0.212334198 |
| GJC3 | gap_junction_protein,_gamma_3_30.2kDa | PDGF.BB_2 RAST_2 IgE_2 | 7 | 7q22.1 | 48 | 0.0009576 | 0.217401842 |

Bonferroni Correction 3.36E-6 [P=0.05]
*No significant difference found as corrected

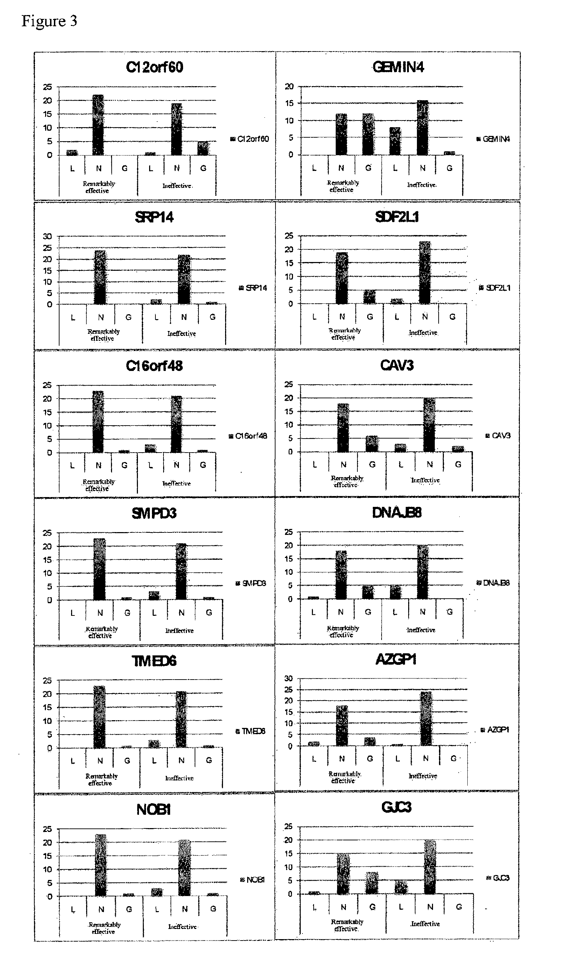

Among analysis models 1), for example, CNV copy number of GJC3 gene was found to show strong correlation with the clinical condition. In particular, all of the cases with the copy number (CN) of 3 ("G" bar in Figure 3) fell in the remarkably effective treatment group, resulting in 30% of the whole remarkably effective treatment group (Figure 3).

[Table 5]

| Gene | Gene name | Serum marker | Chromo some | Chromosomal band | N | P value | R2 value |
|---|---|---|---|---|---|---|---|
| SNORA44 | small_nucleolar_RNA,_H/ACA_box_44 | SCGF.B_5 | 1 | 1p35.3 | 47 | 2.06E-06 | 0.37090163 |
| PAFAH2 | platelet-activating_factor_acetylhydrolase_2,40kDa | IL.16_5 | 1 | 1p36 | 47 | 2.52E-06 | 0.365632168 |
| | | IL.16_6 | | | | 1.66E-06 | 0.376437646 |
| MARK2 | MAP/microtubule_affinity-regulating kinase_2 | SDF.1A_6 | 11 | 11q12-q13 | 47 | 3.24E-06 | 0.358983529 |
| FTH1 | ferritin,_heavy_polypeptide_1 | CTACK_6 | 11 | 11q13 | 47 | 1.29E-06 | 0.382945153 |
| PPFIA1 | protein_tyrosine_phosphatase,_receptor_type,_f_polypeptide_(PTPRF),_interacting_protein_(liprin)_alpha_1 | IL.17_5 | 11 | 11q13.3 | 48 | 8.83E-07 | 0.386101007 |
| SCARNA11 | small_Cajal_body-specific_RNA_11 | SCGF.B_5 | 12 | 12p13.31 | 47 | 1.94E-06 | 0.372476068 |
| NAV3 | neuron_navigator_3 | SCGF.B_5 | 12 | 12q14.3 | 47 | 2.00E-06 | 0.371668677 |
| CUL4A | cullin_4A | IL.17_5 | 13 | 13q34 | 48 | 2.19E-06 | 0.36312568 |
| METHOD | methyltransferase_10_domain_coantaining | SDF.1A_5 | 17 | 17p13.3 | 47 | 2.16E.06 | 0.36962831 |
| ST6GALNAC1 | ST6_(alpha-N-acetyl-neuraminyl-2,3-beta-galactosyl-1,3)-N-acetylgalactosaminide_alpha-2,6-sialyltransferase_1 | GROA_5 | 17 | 17q25.1 | 47 | 3.23E-06 | 0.359052891 |
| C19orf34 | chromosome_19_open_reading_frame_34 | SDF.1A_5 | 19 | 19p13.3 | 47 | 2.40E-06 | 0.366880952 |
| | | IL.17_5 | 19 | 19p13.3 | 48 | 1.83E-06 | 0.367710153 |
| GIGYF2 | GRB10-interacting_GYF_protein_2 | LIF_5 | 2 | 2q37.1 | 47 | 3.05E-06 | 0.360613413 |
| | | IL.17_5 | | | 48 | 8.89E-07 | 0.385926013 |
| _CXCR7_ | chemokine_(C-X-C_motif)_receptor_7 | IL.17_5 | 2 | 2q37.3 | 48 | 1.75E-07 | 0.4251419 |
| ITCH | itchy_E3_ubiquitin_protein_ligase_homolog_(mo use) | IL.17_5 | 20 | 20q11.22-q11.23 | 48 | 2.15E-06 | 0.363600569 |
| SULF2 | sulfatase-2 | IL.16_6 | 20 | 20q12-q13.2 | 47 | 2.33E-06 | 0.367680122 |
| | | SDF.1A_5 | | | | 2.93E-06 | 0.361613012 |
| | | IL.17_5 | | | 48 | 1.84E-06 | 0.367566094 |
| WFDC13 | WAP_four-disulfide_core_domain_13 | IL.17_5 | 20 | 20q13.12 | 48 | 1.95E-06 | 0.366163387 |
| ZNFX1 | zinc_finger,_NFX1-type_containing_1 | IL.17_5 | 20 | 20q13.13 | 48 | 1.77E-06 | 0.36858352 |

| Gene | Gene name | Serum marker | Chromo some | Chromosomal band | N | P value | R2 value |
|---|---|---|---|---|---|---|---|
| HTT | huntingtin | IL.17_5 | 4 | 4p16.3 | 48 | 6.51E-07 | 0.393643249 |
| CEP72 | centrosomal_protein_72kDa | SDF.1A_6 | 5 | 5p15.33 | 49 | 2.59E-06 | 0.352920145 |
| PLEKHG4B | pleckstrin_homology_domain_containing _family _G_(with_RhoGef_domain)_member_4B | SCGF.B_5 | 5 | 5p15.33 | | 1.21E-06 | 0.378300339 |
| | | SDF.1A_5 | | | 48 | 1.78E-06 | 0.368385058 |
| | | SDF.1A_6_ | | | | 1.27E-06 | 0.377084356 |
| | | IL.17_5 | | | 49 | 2.87E.07 | 0.406974024 |
| AGXT2L2 | alanine-glyoxylate_aminotransferase 2-like_2 | LIF_6 | 5 | 5q35.3 | 47 | 2.71E-06 | 0.363654888 |
| | | SDF.1A_5 | | | | 1.69E-06 | 0.375949058 |
| | | SDF.1A_6 | | | | 3.05E-06 | 0.360612552 |
| | | IL.1 7_5 | | | | 2.26E-06 | 0.362326452 |
| | | IL.17_6 | | | | 3.14E-06 | 0.353765562 |
| TYRP1 | tyrosinase-related_protein_1 | SDF.1A_5 | 9 | 9p23 | 47 | 2.97E-06 | 0.361292878 |
| | | IL.4_5 | | | | 2.65E-06 | 0.358242222 |
| | | IL.4_6 | | | 48 | 1.68E-06 | 0.369931555 |
| | | IFN.G_6 | | | | 3.09E-06 | 0.354170759 |
| Bonferroni Correction 3.36E-6 [P=0.05] | | | | | | | |

EP 2 492 353 B1

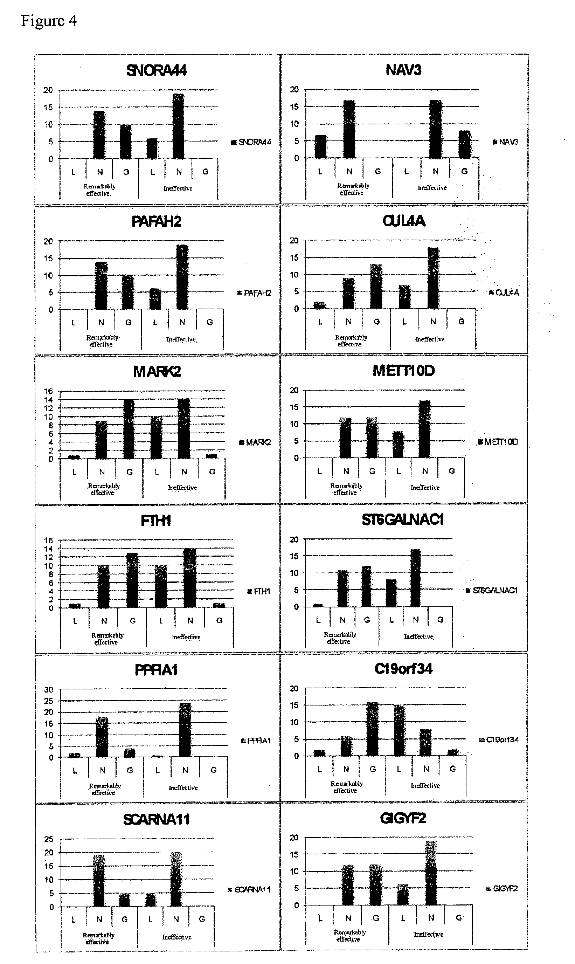

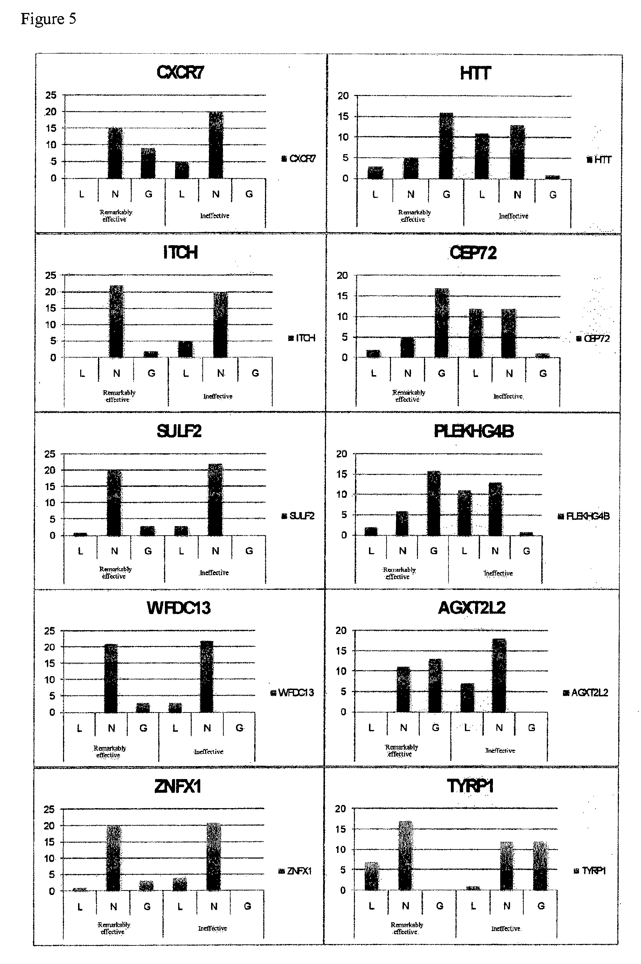

In addition, among analysis model 2), for example, CNV copy numbers of METHOD and CXCR7 genes were found to show strong correlation with the clinical conditions. In particular, all of the cases with the copy number of 3 ("G" bars in Figures 4 and 5) fell in the remarkably effective treatment group, resulting in 50% of the whole remarkably effective treatment group (Figures 4 and 5).

[Table 6-1]

| Gene | Gene name |
|---|---|
| C12orf60 | chromosome_12_open_reading_frame_60 |
| SRP14 | signal_recognition_particle_14kDa_(homologous_Alu_RNA_binding_protein) |
| C16orf48 SMPD3 | chromosome_16_open_reading_frame_48 sphingomyelin_hosphodiesterase_3,_noutral_membrane_(neutral_sphingomyelinase_II) |
| TMED6 | transmembrane_emp24_protein_transport_domain_containing_6 |
| NOB1 GEMIN4 SDF2L1 | NIN1/RPN12_binding_protein_1_homolog_(S. cerevisiae) gem_(nuclear_organelle)_associated_protein_4 stromal_cell-derived_factor_2-like_1 |
| CAV3 | caveolin_3 |
| DNAJB8 AZGP1 | DnaJ_(Hsp40)_homolog,_subfamily_B,_member_8 alpha-2-glycoprotein_1,_zinc-binding |
| GJC3 | gap_junction_protein,_gamma_3,_30.2kDa |
| SNORA44 | small_nucleolar_RNA,_H/ACA_box_44 |
| PAFAH2 | platelet-activating_factor_acetylhydrolase_2,_40kDa |
| MARK2 | MAP/microtubule_affinity-regulating_kinase_2 |
| FTH1 | ferritin,_heavy_polypeptide_1 |
| PPFIA1 | protein_tyrosine_phosphatase,_receptor_type,_f_polypeptide_(PTPRF),_interacti ng_protein_ (liprin),_alpha_1 |
| SCARNA11 | small_Cajal_body-specific_RNA_11 |
| NAV3 | neuron_navigator_3 |
| CUL4A | cullin_4A |
| METT10D | methyltransferase_10_domain_containing |
| ST6GALNAC1 | ST6_(alpha-N-acetyl-neuraminyl-2,3-beta-galactosyl-1,3)-N-acetylgalactosamini de_alpha-2,6- sialyltransferase_1 |
| C19orf34 | chromosome_19_open_reading_frame_34 |
| GIGYF2 | GRB10_interacting_GYF_protein_2 |
| CXCR7 | chemokine_(C-X-C_motif)_receptor_7 |
| ITCH | itchy_E3_ubiquitin_protein_ligase_homolog_(mouse) |
| SULF2 | sulfatase_2 |
| WFDC13 | VAP_four-disulfide_core_domain_13 |

[Table 6-2]

| | |
|---|---|
| ZNFX1 | zinc_finger,_NFX1-type_containing_1 |
| HTT | huntingtin |
| CEP72 | centrosomal_protein_72kDa |
| PLEKHG4B | pleckstrin_homology_domain_containing,_family_G_(with_RhoGef_domain)_member_4 B |

(continued)

| | |
|---|---|
| AGXT2L2 | alanine-glyoxylate_aminotransferase_2-like_2 |
| TYRP1 | tyrosinase-related_protein_1 |

Furthermore, for each gene, looking at the CNV copy numbers of patients whose treatments were remarkably effective (24 cases) or ineffective (25 cases), the number of patients with CNV copy number of 1 for the gene was compared with the number of patients with CNV copy number of 3. Then, the CNV copy number for the gene that appears more often in the remarkably effective group was scored +1 while the CNV copy number that appears more often in the ineffective group was scored - 1. Furthermore, when the difference between the number of patients with CNV copy number of 1 and the number of patients with CNV copy number of 3 for the gene is equal to or less than 1, it was scored 0.

The results obtained by applying this scoring to each patient are shown in Table 7. The scores for each patient were summed, and distribution and the summed score for each patient are shown in Table 8.

[Table7]

| Judgment | | C12orf60 | SRP14 | C16orf48 | SMPD3 | TMED6 | NOB1 | GEMIN4 | SDF2L1 | CAV3 | DNAJB8 | AZGP1 |
|---|---|---|---|---|---|---|---|---|---|---|---|---|
| | CNV=1 | 0 | -1 | -1 | -1 | -1 | -1 | -1 | 0 | -1 | -1 | 0 |
| | CNV=3 | -1 | 0 | 0 | 0 | 0 | 0 | 1 | 1 | 1 | 1 | 1 |

| Group | No. | C12orf60 | SRP14 | C16orf48 | SMPD3 | TMED6 | NOB1 | GEMIN4 | SDF2L1 | CAV3 | DNAJB8 | A2GP1 |
|---|---|---|---|---|---|---|---|---|---|---|---|---|
| Remarkably effective group | 1 | | | | | | | 1 | | | | |
| | 2 | | | | | | | 1 | 1 | | | 1 |
| | 3 | | | | | | | 1 | 1 | | | |
| | 4 | | | | | | | | | | | 1 |
| | 5 | | | | | | | | | -1 | | |
| | 6 | | | | | | | | | | | 1 |
| | 7 | | | | | | | | | | | |
| | 8 | | | | | | | 1 | | | | |
| | 9 | | | | | | | | | | | |
| | 10 | | | | | | | 1 | | | 1 | |
| | 11 | | | | | | | 1 | 1 | | 1 | 0 |
| | 12 | | | | | | | | | | | |
| | 13 | | | | | | | 1 | | 1 | | |
| | 14 | | | 0 | 0 | 0 | 0 | 1 | 1 | | | 1 |
| | 15 | | | | | | | 1 | | 1 | | |
| | 16 | 0 | | | | | | | | 1 | 1 | |
| | 17 | | | | | | | 1 | | 1 | | |
| | 18 | | | | | | | 1 | | 1 | | |
| | 19 | | | | | | | | | | | |
| | 20 | | | | | | | | 1 | | | 0 |
| | 21 | 0 | | | | | | | | | | |
| | 22 | | | | | | | | | | | |
| | 23 | | | | | | | | | | | |
| | 24 | | | | | | | | | 1 | | |
| Ineffective group | 25 | | | | | | | | | | | |
| | 26 | | | | | | | | | | | |
| | 27 | | | | | | | | | | | |
| | 28 | | | | | | | | | | | |
| | 29 | | | | | | | | | | | |
| | 30 | | | | | | | 1 | | | | |
| | 31 | | -1 | | | | | -1 | | | -1 | -1 |
| | 32 | | | | | | | -1 | | | -1 | |
| | 33 | | | | | | | | | | | |
| | 34 | | | | | | | | | | | |
| | 35 | -1 | -1 | -1 | -1 | -1 | -1 | -1 | 0 | -1 | -1 | |
| | 36 | | | | | | | | | | | |
| | 37 | | | -1 | -1 | -1 | -1 | | | | | |
| | 38 | | | | | | | | | | | |
| | 39 | | | | | | | | | | | |
| | 40 | -1 | | | | | | -1 | | | 1 | -1 |
| | 41 | -1 | | -1 | -1 | -1 | -1 | -1 | | | -1 | |
| | 42 | | | | | | | | | | | 0 |
| | 43 | | | | | | | | | | | |
| | 44 | | | | | | | | | | | |
| | 45 | | | 0 | 0 | 0 | 0 | -1 | | | 1 | |
| | 46 | | | | | | | | | | | |
| | 47 | -1 | 0 | | | | | -1 | | | | |
| | 48 | 0 | -1 | | | | | -1 | | 0 | -1 | |
| | 49 | | | | | | | | | | | |

| GJC3 | SNORA44 | PAFAH2 | MARK2 | FTH1 | PPFIA1 | SCARNA11 | NAV3 | CUL4A | METT10D | ST6GALNAC1 | C19orf34 |
|---|---|---|---|---|---|---|---|---|---|---|---|
| -1 | -1 | -1 | -1 | -1 | 0 | -1 | 1 | -1 | -1 | -1 | -1 |
| 1 | 1 | 1 | 1 | 1 | 1 | 1 | -1 | 1 | 1 | 1 | 1 |

| GIGYF2 | CXCR7 | ITCH | SULF2 | WFDC13 | ZNFX1 | HTT | CEP72 | PLEKHG4B | AGXT2L2 | TYRP1 |  |
|---|---|---|---|---|---|---|---|---|---|---|---|
| -1 | -1 | -1 | 0 | -1 | -1 | -1 | -1 | -1 | -1 | 1 |  |
| 1 | 1 | 0 | 1 | 1 | 1 | 1 | 1 | 1 | 1 | -1 |  |

| GIGYF2 | CXCR7 | ITCH | SULF2 | WFDC13 | ZNFX1 | HTT | CEP72 | PLEKHG4B | AGXT2L2 | TYRP1 | SUM |
|---|---|---|---|---|---|---|---|---|---|---|---|
|  |  |  |  |  |  |  | 1 | 1 |  |  | 7 |
|  | 1 | 1 |  |  |  |  | 1 | 1 | 1 |  | 20 |
|  | 1 | 1 |  |  |  |  | 1 | 1 |  |  | 13 |
|  |  |  |  |  |  |  |  |  |  |  | 1 |
|  |  |  | 0 |  | -1 | -1 | -1 | -1 |  |  | -9 |
|  |  |  |  |  |  | -1 |  |  |  |  | -1 |
|  |  |  |  |  |  |  |  |  |  |  | 0 |
|  |  |  |  |  |  | 1 | 1 |  |  |  | 9 |
|  |  |  |  |  |  | 1 |  |  |  |  | -1 |
|  | 1 | -1 |  |  |  |  |  |  | 1 | 1 | 17 |
|  | 1 |  | 0 | 1 | 1 | 1 |  |  |  |  | 22 |
|  |  |  |  |  |  |  |  |  |  |  | 0 |
|  | 1 | 1 |  |  |  | 1 | 1 | 1 |  |  | 19 |
|  | 1 | 1 |  |  |  | 1 | 1 |  |  |  | 21 |
|  | 1 | 1 |  |  |  |  |  |  |  |  | 19 |
|  | 1 | 1 |  |  |  |  |  |  |  |  | 17 |
|  | 1 | 1 |  |  |  |  |  |  |  |  | 19 |
|  | 1 | 1 |  |  |  |  |  |  |  |  | 17 |
|  |  |  |  | 1 | 1 | 1 | 1 |  |  |  | 11 |
|  | 1 | 1 |  |  |  | 1 |  |  | 1 |  | 10 |
|  |  |  |  |  |  | 1 |  | 1 | 1 |  | 9 |
|  |  |  |  |  |  | -1 | -1 |  |  |  | 2 |
|  |  |  |  |  |  | -1 | -1 |  |  |  | -3 |
|  | 1 | 0 | 1 | 1 | 1 | 1 | 1 | 1 | 1 | 1 | 17 |
|  |  |  |  |  |  |  |  |  |  |  | 0 |
|  |  |  |  |  |  |  |  |  |  |  | 0 |
|  |  |  |  |  |  | 1 | 1 | 1 |  | 1 | 7 |
|  |  |  |  |  |  |  |  |  |  |  | 0 |
|  |  |  |  |  |  |  |  |  |  |  | 0 |
|  |  |  |  |  |  |  |  |  |  |  | 2 |
|  | -1 | -1 |  |  |  | -1 | -1 |  | -1 | -1 | -16 |
|  |  |  |  |  |  | -1 | -1 | -1 |  |  | -11 |
|  | -1 | -1 |  |  |  | -1 | -1 | -1 |  |  | -6 |
|  |  |  |  |  |  |  |  |  |  |  | 0 |
|  | -1 | -1 | -1 | 0 | -1 | -1 | -1 |  | -1 | -1 | -30 |
|  |  |  |  |  |  |  |  |  |  |  | 0 |
|  |  |  |  |  |  | -1 |  |  |  | -1 | -9 |
|  |  |  |  |  |  |  |  |  |  |  | 0 |
|  |  |  |  |  |  |  | -1 | -1 |  | -1 | -5 |
|  | -1 | -1 | -1 |  |  | -1 | -1 | -1 | -1 | -1 | -20 |
|  | -1 | -1 |  |  |  | -1 | -1 | -1 | -1 | -1 | -25 |
|  |  |  |  |  |  |  |  |  |  | -1 | -11 |
|  | -1 |  |  |  |  |  |  |  |  |  | -1 |
|  |  |  |  |  |  |  |  |  |  |  | -1 |
|  |  | -1 | 0 | -1 | -1 | -1 | -1 | -1 |  | -1 | -13 |
|  |  |  |  |  |  |  | -1 | -1 |  | -1 | -6 |
|  |  |  |  |  | -1 | -1 | -1 | -1 |  | -1 | -17 |
|  | -1 | -1 | 0 | -1 | -1 | -1 | -1 | -1 | -1 | -1 | -22 |
|  |  |  |  |  |  |  |  |  |  | -1 | -4 |

[Table 8]

| Score | Count(people) | |
|---|---|---|
| | Remarkably effective | Ineffective |
| 33 | 0 | 0 |
| 32 | 0 | 0 |
| 31 | 0 | 0 |
| 30 | 0 | 0 |
| 29 | 0 | 0 |
| 28 | 0 | 0 |
| 27 | 0 | 0 |
| 26 | 0 | 0 |
| 25 | 0 | 0 |
| 24 | 0 | 0 |
| 23 | 0 | 0 |
| 22 | 1 | 0 |
| 21 | 1 | 0 |
| 20 | 1 | 0 |
| 19 | 3 | 0 |
| 18 | 0 | 0 |
| 17 | 4 | 0 |
| 16 | 0 | 0 |
| 15 | 0 | 0 |
| 14 | 0 | 0 |
| 13 | 1 | 0 |
| 12 | 0 | 0 |
| 11 | 1 | 0 |
| 10 | 1 | 0 |
| 9 | 2 | 0 |
| 8 | 0 | 0 |
| 7 | 1 | 1 |
| 6 | 0 | 0 |
| 5 | 0 | 0 |
| 4 | 0 | 0 |
| 3 | 0 | 0 |
| 2 | 1 | 1 |
| 1 | 1 | 0 |
| 0 | 2 | 7 |

(cont.)

| | | |
|---|---|---|
| -1 | 2 | 2 |
| -2 | 0 | 0 |
| -3 | 1 | 0 |
| -4 | 0 | 1 |
| -5 | 0 | 1 |
| -6 | 0 | 2 |
| -7 | 0 | 0 |
| -8 | 0 | 0 |
| -9 | 1 | 1 |
| -10 | 0 | 0 |
| -11 | 0 | 2 |
| -12 | 0 | 0 |
| -13 | 0 | 1 |
| -14 | 0 | 0 |
| -15 | 0 | 0 |
| -16 | 0 | 1 |
| -17 | 0 | 1 |
| -18 | 0 | 0 |
| -19 | 0 | 0 |
| -20 | 0 | 1 |
| -21 | 0 | 0 |
| -22 | 0 | 1 |
| -23 | 0 | 0 |
| -24 | 0 | 0 |
| -25 | 0 | 1 |
| -26 | 0 | 0 |
| -27 | 0 | 0 |
| -28 | 0 | 0 |
| -29 | 0 | 0 |
| -30 | 0 | 1 |
| -31 | 0 | 0 |
| -32 | 0 | 0 |
| -33 | 0 | 0 |
| Total | 24 | 25 |

| | ~-1 | 0 | 1~ | Total(people) |
|---|---|---|---|---|
| Rernarkably effective | 4 | 2 | 18 | 24 |
| Ineffective | 16 | 7 | 2 | 25 |

Target genes to be selected were defined as genes having difference in the numbers of people between the remarkably effective group and the ineffective group of 2 or more for CNV of 1 or 3 according to the following criteria.

(a) Remarkably effective when CNV is 1
(b) Remarkably effective when CNV is 3
(c) Ineffective when CNV is 1
(d) Ineffective when CNV is 3

Genes that fall in the above-described criteria are shown in Table 9. In Table 9, circles are marked on the right side for the genes that fall in the above-described criteria.

[Table 9]

| (RE): Remarkably Effective (IE): Ineffective | | | | | | | | (a) CNV1 (RE) | (b) CNV3 (RE) | (c) CNV1 (IE) | (d) CNV3 (IE) |
|---|---|---|---|---|---|---|---|---|---|---|---|
| 1(RE) | 1(IE) | 1(RE)-1(IE) | Gene name | 3(RE) | 3(IE) | 3(RE)-3(IE) | Gene name | | | | |
| 2 | 1 | 1 | C12orf60 | 0 | 5 | -5 | C12orf60 | | | | ○ |
| 0 | 2 | -2 | SRP14 | 0 | 1 | -1 | SRP14 | | | ○ | |
| 0 | 3 | -3 | C16orf48 | 1 | 1 | 0 | C16orf48 | | | ○ | |
| 0 | 3 | -3 | SMPD3 | 1 | 1 | 0 | SMPD3 | | | ○ | |
| 0 | 3 | -3 | TMED6 | 1 | 1 | 0 | TMED6 | | | ○ | |
| 0 | 3 | -3 | NOB1 | 1 | 1 | 0 | NOB1 | | | ○ | |
| 0 | 8 | -8 | GEMIN4 | 12 | 1 | 11 | GEMIN4 | | ○ | ○ | |
| 0 | 2 | -2 | SDF2L1 | 5 | 0 | 5 | SDF2L1 | | ○ | ○ | |
| 0 | 3 | -3 | CAV3 | 6 | 2 | 4 | CAV3 | | ○ | ○ | |
| 1 | 5 | -4 | DNAJB8 | 5 | 0 | 5 | DNAJB8 | | ○ | ○ | |
| 2 | 1 | 1 | AZGP1 | 4 | 0 | 4 | AZGP1 | | ○ | | |
| 1 | 5 | -4 | GJC3 | 8 | 0 | 8 | GJC3 | | ○ | ○ | |
| | | | | | | | | | | | |
| 1(RE) | 1(IE) | 1(RE)-1(IE) | Gene name | 3(RE) | 3(IE) | 3(RE)-3(IE) | Gene name | | | | |
| 0 | 6 | -6 | SNORA44 | 10 | 0 | 10 | SNORA44 | | ○ | ○ | |
| 0 | 6 | -6 | PAFAH2 | 10 | 0 | 10 | PAFAH2 | | ○ | ○ | |
| 1 | 10 | -9 | MARK2 | 14 | 1 | 13 | MARK2 | | ○ | ○ | |
| 1 | 10 | -9 | FTH1 | 13 | 1 | 12 | FTH1 | | ○ | ○ | |
| 2 | 1 | 1 | PPFIA1 | 4 | 0 | 4 | PPFIA1 | | ○ | | |
| 0 | 5 | -5 | SCARNA11 | 5 | 0 | 5 | SCARNA11 | | ○ | ○ | |
| 7 | 0 | 7 | NAV3 | 0 | 8 | -8 | NAV3 | ○ | | | ○ |
| 2 | 7 | -5 | CUL4A | 13 | 0 | 13 | CUL4A | | ○ | ○ | |
| 0 | 8 | -8 | METT10D | 12 | 0 | 12 | METT10D | | ○ | ○ | |

(continued)

| 1(RE) | 1(IE) | 1(RE)-1(IE) | Gene name | 3(RE) | 3(IE) | 3(RE)-3(IE) | Gene name | | | | |
|---|---|---|---|---|---|---|---|---|---|---|---|
| 1 | 8 | -7 | ST6GALNAC1 | 12 | 0 | 12 | ST6GALNAC1 | | | | |
| 2 | 15 | -13 | C19orf34 | 16 | 2 | 14 | C19orf34 | | ○ | ○ | |
| 0 | 6 | -6 | GIGYF2 | 12 | 0 | 12 | GIGYF2 | | ○ | ○ | |
| 0 | 5 | -5 | CXCR7 | 9 | 0 | 9 | CXCR7 | | ○ | ○ | |
| 0 | 5 | -5 | ITCH | 2 | 0 | 2 | ITCH | | ○ | ○ | |
| 1 | 3 | -2 | SULF2 | 3 | 0 | 3 | SULF2 | | ○ | ○ | |
| 0 | 3 | -3 | WFDC13 | 3 | 0 | 3 | WFDC13 | | ○ | ○ | |
| 1 | 4 | -3 | ZNFX1 | 3 | 0 | 3 | ZNFX1 | | ○ | ○ | |
| 3 | 11 | -8 | HTT | 16 | 1 | 15 | HTT | | ○ | ○ | |
| 2 | 12 | -10 | CEP72 | 17 | 1 | 16 | CEP72 | | ○ | ○ | |
| 2 | 11 | -9 | PLEKHG4B | 16 | 1 | 15 | PLEKHG4B | | ○ | ○ | |
| 0 | 7 | -7 | AGXT2L2 | 13 | 0 | 13 | AGXT2L2 | | ○ | ○ | |
| 7 | 1 | 6 | TYRP1 | 0 | 12 | -12 | TYRP1 | ○ | | | ○ |

As a result, the allergen immunotherapy was found to have a tendency to be effective for NAV3 and TYRP1 when the copy number was 1 while the allergen immunotherapy was found to have a tendency to be effective for AGXT2L2, AZGP1, CAV3, CEP72, CUL4A, CXCR7, C19orf34, DNAJB8, FTH1, GEMIN4, GIGYF2, GJC3, ITCH, HTT, MARK2, METT10D, PAFAH2, PLEKHG4B, PPFIA1, SCARNA11, SDF2L1, SNORA44, ST6GALNAC1, SULF2, WFDC13 and ZNFX1 when the copy number was 3.

On the other hand, the allergen immunotherapy was found to have a tendency to be ineffective for AGXT2L2, CAV3, CEP72, CUL4A, CXCR7, C16orf48, C19orf34, DNAJB8, FTH1, GEMIN4, GIGYF2, GJC3, HTT, ITCH, MARK2, METT10D, NOB1, PAFAH2, PLEKHG4B, SCARNA11, SDF2L1, SMPD3, SNORA44, SRP14, ST6GALNAC1, SULF2, TMED6, WFDC13 and ZNFX1 when the copy number was 1 while the allergen immunotherapy was found to have a tendency to be ineffective for C12orf60, NAV3 and TYRP1 when the copy number was 3.

Example 2

Search for genes that contribute to assessment of efficacy with higher probability

According to the present example, multiple regression analysis was performed for the purpose of searching for genes necessary for carrying out the judgment of the efficacy with higher probability.

A relational expression between a certain variable y (referred to as a criterion variable or a dependent variable) and variables x1, x2, ...xp that are considered to influence variable y (referred to as explanatory variables or independent variables) was obtained, based on which y value can be predicted from values x1, x2, ...xp or the contribution level of each x upon such prediction can be assessed. Such analysis is referred to as regression analysis. In particular, the analysis is called a multiple regression analysis when there are two or more explanatory variables.

According to the present example, for the above-described 34 genes, a multiple regression analysis was conducted based on a general linear model having binomial dependent variable y representing remarkable effectiveness and ineffectiveness and trinomial explanatory variable representing the copy number of each gene where 2 copies (CNV=2) being the baseline, increase therefrom (CNV=3) as 1 and decrease therefrom (CNV=1) as -1, thereby selecting an AIC model using a backward elimination algorithm (step set value: 10,000). Here, an AIC model ("Akaike's Information Criterion" model) refers to a statistical indicator that is frequently used worldwide and that was developed for the purpose of model selection, where a model with the minimum AIC is selected given a plurality of models for an identical data set.

The multiple regression analysis was conducted using R-2.9 program software (R Development Core Team).

The results are shown in Table 10.

[Table 10]

**Relationship between analysis model and influence of the gene**

| Case Ctrl vs Genes | Df | Sum of Sq | RSS | AIC | F-value | Pr(F) | | β(Regression coefficient) |
|---|---|---|---|---|---|---|---|---|
| | | <none> | 3.185 | -97.936 | | | | 0.3536 |
| -TYRP1 | 1 | 0.162 | 3.346 | -97.512 | 1.5722 | 0.2193 | | -0.2148 |
| -MARK2 | 1 | 0.223 | 3.408 | -96.626 | 2.1666 | 0.1511 | | 0.7405 |
| -NAV3 | 1 | 0.268 | 3.453 | -95.975 | 2.6101 | 0.1163 | | 0.3163 |
| -CAV3 | 1 | 0.36 | 3.544 | -94.696 | 3.4994 | 0.0709 | | -0.2938 |
| -ZNFX1 | 1 | 0.392 | 3.576 | -94.255 | 3.8111 | 0.06 | | -0.5182 |
| -AGXT2L2 | 1 | 0.425 | 3.61 | -93.793 | 4.1408 | 0.0505 | | 0.4164 |
| -FTH1 | 1 | 0.52 | 3.704 | -92.531 | 5.0573 | 0.0318 | * | -1.0428 |
| -GEMIN4 | 1 | 0.532 | 3.717 | -92.369 | 5.1774 | 0.0299 | * | -0.6616 |
| -SDF2L1 | 1 | 0.619 | 3.804 | -91.238 | 6.0219 | 0.0199 | * | -0.7666 |
| -HTT | 1 | 0.659 | 3.844 | -90.722 | 6.414 | 0.0166 | * | -0.5237 |
| -C19orF34 | 1 | 0.743 | 3.928 | -89.666 | 7.2288 | 0.0114 | * | 0.5029 |
| -ITCH | 1 | 0.789 | 3.974 | -89.089 | 7.6818 | 0.0093 | ** | -0.8697 |
| -AZGP1 | 1 | 0.793 | 3.977 | -89.048 | 7.7139 | 0.0092 | ** | 0.6013 |
| -PLEKHG4B | 1 | 0.818 | 4.003 | -88.736 | 7.9609 | 0.0083 | ** | -1.0924 |
| -CEP72 | 1 | 1.004 | 4.189 | -86.51 | 9.7721 | 0.0038 | ** | 1.3694 |
| -WFDC13 | 1 | 1.035 | 4.22 | -86.15 | 10.0729 | 0.0034 | ** | 1.1209 |
| -METT10D | 1 | 1.108 | 4.293 | -85.308 | 10.7846 | 00025 | ** | 0.9797 |

As a result, after the process of 18-stage stepwise selection of AIC optimal model, 17 genes with AIC = 97.94 were found. Hence, use of the above-mentioned 17 genes for the judgment of the remarkably effective/ineffective groups was found to be an optimal analysis model. The regression coefficient β shows the relationship between the copy number and efficacy. Positive (+) β value in the analysis model means that the increase in the copy number (CN=3) upon analysis of the remarkably effective and ineffective groups is likely to be related to the remarkably effective group whereas negative (-) β means that the decrease in the copy number is likely to be related to the ineffective group.

Example 3

mRNA analysis was performed using mRNA derived from the basophil fractionated from the blood sample drawn at blood drawing point 7 by FACS. In addition, CNV analysis was carried out for each patient group (22 patients from the remarkably effective treatment group and 22 patients from the therapeutically ineffective group (total of 44 patients)). The analysis model was "efficacy assessment (alleviation) in 2008, CNV: mRNA (blood drawing point 7 after the treatment)". Other than the above-described points, the analysis was carried out in the same manner as Example 1.

Specifically, for 13,792 genes intertwined with gene symbols based on mRNA data and CNV data, a linear model regression analysis was conducted using three parameters, i.e., serological test term, mRNA value and CNV value of the above-mentioned analysis model as explanatory variables and the value of efficacy judgment of the cedar pollen extract trial in the spring of 2008 as dependent variables.

P values and contribution levels (corrected R2 values) were calculated for about 1.49 million analyses as the analysis model. Sorting was conducted by filtering using contribution level of the analysis model to the regression line as criteria. Thereafter, relationship with P value was assessed as Bonferroni corrected P value.

The resulting analysis model and each explanatory variable were examined in detail in terms of the association between the clinical serum marker value, the mRNA value or the CNV value and the clinical phenotype, thereby obtaining a final candidate model.

Specifically, with respect to each grade of the analysis model (stages 1 to 3), R-2.9 program was used to calculate R2 value as the contribution level and P value, for a combination in which any one term is modified in proportion to the measure of other term in a non-additive association, as an interaction term of three parameter terms, i.e., (a) mRNA gene Log2 strength, (b) relative Log2 ratio of the CNV segment on the gene and (c) serological test terms determined before and after the mRNA collection/blood drawing point. Here, the value from blood drawing 6 was made to correspond to the analysis model.

After the calculation of the statistic values, first-stage filtering was performed to extract combinations as candidate combinations of R2 > 0.25 and P value < 0.00000363. As a result, 254 combinations were obtained. Among these combinations, combinations were obtained by second-stage filtering in which the results of copy number judgment obtained in the CNV analysis were once again assessed with respect to the CNV copy number of the gene.

Thus, the present analysis model and the serological test terms were used for a general linear regression model analysis to extract significant serological test term (P value < 0.05) in this model analysis. As a result, 21 genes were obtained with 254 combinations (see Tables 11 and 12).

[Table 11]

| Gene Name | Serum marker 6th Blood drawing | P value * | Corrected r2 value** [Contribution level] |
|---|---|---|---|
| CEDC127 | CTACK | 1.29E-06 | 0.3075297 |
| | IFN.A2 | 1.21E-06 | 0.310647 |
| | LIF | 2.29E-06 | 0.3837115 |
| | TRAIL | 2.45E-06 | 0.318395 |
| | SCF | 4.63E-07 | 0.3765896 |
| | SCGF.B | 8.05E-08 | 0.3258075 |
| | SDF.1A | 3.16E-07 | 0.3258505 |
| NCAM2 | IL.16 | 1.14E-06 | 0.2813149 |
| | CTACK | 8.15E-07 | 0.2604172 |
| | IFN.A2 | 2.52E-07 | 0.2963275 |
| | SCGF.B | 3.24E-06 | 0.2687564 |
| | SDF.1A | 3.76E-07 | 0.2868119 |
| | IL.4 | 3.55E-07 | 0.3002483 |
| | IFN.G | 3.48E-07 | 0.3138113 |

(continued)

| Gene Name | Serum marker 6th Blood drawing | P value * | Corrected r2 value** [Contribution level] |
|---|---|---|---|
| PCDH17 | 1L.16 | 2.77E-06 | 0.3463412 |
| | CTACK | 6.92E-07 | 0.3508945 |
| | SCGF.B | 1.06E-06 | 0.3310659 |
| | SDF.1A | 1.57E-06 | 0.3600703 |
| | IP.10 | 2.93E-07 | 0.3492307 |
| C14orf180 | CTACK | 2.66E-06 | 0.2533242 |
| | GROA | 3.11E-06 | 0.3073281 |
| | 1FN.A2 | 2.36E-07 | 0.3666799 |
| | SCGF.B | 3.05E-06 | 0.2867621 |
| | SDF.1A | 7.57E-07 | 0.3960845 |
| CHODL | IFN.A2 | 1.82E-06 | 0.296123 |
| | SCGF.B | 3.46E-06 | 0.2528756 |
| | SDF.1A | 3.24E-06 | 0.2693426 |
| | IL.4 | 2.16E-06 | 0.2695358 |
| | IFN.G | 2.06E-06 | 0.2713536 |
| SIVA1 | CTACK | 2.66E-06 | 0.2803814 |
| | GROA | 3.11E-06 | 0.4118834 |
| | 1FN.A2 | 2.36E-07 | 0.3924536 |
| | SCGF.B | 3.05E-06 | 0.3543863 |
| | SDF.1A | 7.57E-07 | 0.4137064 |
| TNFRSF14 | CTACK | 9.24E-07 | 0.2722765 |
| | IFN.A2 | 6.95E-07 | 0.2955877 |
| | SCF | 2.49E-06 | 0.3615561 |
| | SCGF.B | 8.12E-07 | 0.3256602 |
| | SDF.1A | 1.71E-06 | 0.2925794 |
| AHNAK2 | CTACK | 1.62E-06 | 0.3082199 |
| | GROA | 2.05E-06 | 0.3626634 |
| | 1FN.A2 | 9.30E-07 | 0.307904 |
| | SGGF.B | 2.18E-06 | 0.3541045 |
| C14orF79 | CTACK | 1.62E-06 | 0.2183435 |
| | GROA | 2.05E-06 | 0.3336502 |
| | IFN.A2 | 9.30E-07 | 0.292211 |
| | SCGF.B | 2.18E-06 | 0.3084159 |
| LOC25845 (LRRC14B) | CTACK | 4.31E-07 | 0.2945138 |
| | 1FN.A2 | 4.50E-07 | 0.3275292 |
| | SCGF.B | 2.09E-06 | 0.2520615 |
| | SDF.1A | 7.61E-07 | 0.3274352 |
| PLD4 | CTACK | 1.62E-06 | 0.2732138 |
| | GROA | 2.05E-06 | 0.2801979 |
| | IFN.A2 | 9.30E-07 | 0.3069721 |
| | SCGF.B | 2.18E-06 | 0.2869556 |
| GPR132 | CTACK | 1.62E-06 | 0.297574 |
| | GROA | 2.05E-06 | 0.2691575 |
| | IFN.A2 | 9.30E-07 | 0.2601031 |

(continued)

| Gene Name | Serum marker 6th Blood drawing | P value * | Corrected r2 value** [Contribution level] |
|---|---|---|---|
| LOC389257 | TRAIL | 2.45E-06 | 0.2564211 |
| | SCF | 4.63E-07 | 0.2802226 |
| | SCGF.B | 8.05E-08 | 0.3102645 |
| BRF1 | CTACK | 1.62E-06 | 0.2503298 |
| | IFN.A2 | 9.30E-07 | 0.2765277 |
| BTG3 | IFN.G | 3.43E-06 | 0.2513725 |
| | IP.10 | 6.82E-07 | 0.3204643 |
| DIAPH3 | SCGF.B | 8.03E-07 | 0.2637675 |
| | IFN.G | 2.46E-06 | 0.2646007 |
| GBA3 | IFN.A2 | 2.29E-06 | 0.2611828 |
| | IP.10 | 1.38E-07 | 0.2665606 |
| IFRD1 | IFN.A2 | 1.19E-06 | 0.3225129 |
| | IP.10 | 1.28E-06 | 0.277858 |
| KCNT2 | IL.4 | 2.64E-06 | 0.2651524 |
| | IFN.G | 1.83E-06 | 0.2613099 |
| THOC7 | IFN.A2 | 2.33E-06 | 0.2570067 |
| | IP.10 | 2.14E-06 | 0.3409871 |
| TMEM168 | IFN.A2 | 1.19E-06 | 0.3046113 |
| | IP.10 | 1.28E-06 | 0.2508919 |

*P value adjusted by Bonferroni correction [P < 0.05]
**r2 value > 0.25

Correlations between the CNV copy number of each gene and the clinical condition are shown in Tables 6 to 8.

[Table 12]

| Gene Name | Standard nomenclature |
|---|---|
| CCDC127 | "coiled-coil domain containing 127" |
| NCAM2 | "neural cell adhesion molecule 2" |
| PCDH17 | "'protocadherin 17 |
| C14orf180 | "chromosome 14 open reading frame 180" |
| CHODL | "chondrolectin" |
| SIVA1 | "apoptosis-inducing factor |
| TNFRSF14 | "tumor necrosis factor receptor superfamily, member 14 (herpesvirus entry mediator)" |
| AHNAK2 | "nucleoprotein 2" |
| C14orf79 | "chromosome 14 open reading frame 79" |
| LOC25845 | "-" |
| PLD4 | "phospholipase D family, member 4" |
| GPR132 | "G protein-coupled receptor 132" |
| LOC389257 | "leucine rich repeat containing 14B" |
| BRF1 | "BRF1 homolog, subunit of RNA polymerase III transcription initiation factor IIIB (S. cerevisiae)" |
| BTG3 | "BTG family, member 3" |
| DIAPH3 | "diaphanous homolog 3 (Drosophila)" |
| GBA3 | "glucosidase, beta, acid 3 (cytosolic)" |
| IFRD1 | "interferon-related developmental regulator 1" |
| KCNT2 | "potassium channel, subfamily T, member 2" |
| THOC7 | "THO complex 7 homolog (Drosophila)" |

(continued)

| Gene Name | Standard nomenclature |
|-----------|------------------------|
| TMEM168 | "transmembrane protein 168" |

**Targets of analysis**

**Remarkably effective: 22 patients**
**Ineffective: 22 patients**

Other than the above-described genes, the following genes were also suggested of their significance. Therefore, in the present example, 39 genes consisting of the above-mentioned 21 genes found to have significance as well as the following 13 genes suggested to have significance (Table 13) were analyzed.

[Table 13]

| Gene Name | Standard nomenclature | Accession Number |
|-----------|------------------------|------------------|
| ADSSL1 | "adenylosuccinate synthase like 1" | NM_152328 |
| BST1 | "bone marrow stromal cell antigen 1" | NM_004334 |
| C7orf53 | "chromosome 7 open reading frame 53" | NM_001134468 |
| CD38 | "CD38 molecule" | NM_001775 |
| DCUN1D1 | " DCN1, defective in cullin neddylation 1, domain containing 1" | NM_020640 |
| FGFBP1 | "fibroblast growth factor binding protein 1" | NM_005130 |
| FOXP2 | "forehead box P2" | NM_001172766 |
| GLRB | "glycine receptor, beta" | NM_000824 |
| GTF2B | "general transcription factor ITB" | NM_001514 |
| HSP90AB2P | "heat shock protein 90kDa alpha (cytosolic), class B member 2 (pseudogene)" | NR_003132 |
| MCCC1 | "methylcrotonoyl-CoA carboxylase 1 (alpha)" | NM_020168 |
| MDFIC | "MyoD family inhibitor domain containing" | NO_001166345 |
| ODF2L | "outer dense fiber of sperm tails 2-like" | NM_001007022 |
| PDGFC | "platelet derived growth factor C" | NM_016205 |
| sep15 | "15 kDa selenoprotein" | NM_004261 |
| SH3GLB1 | "SH3-domain GRB2-like endophilin B1" | NM_016009 |
| TDRD3 | "tudor domain containing 3" | NM_001146070 |
| YEATS2 | "YEATS domain containing 2" | NM_018023 |

Similar to Example 1, focusing on the CNV copy numbers of the patients with remarkable treatment effectiveness (22 cases) and patients with ineffectiveness (22 cases) for each gene, the number of patients having CNV copy number of 1 was compared with the number of patients having CNV copy number of 3 for the gene so as to carry out scoring of the CNV copy numbers. The results obtained by applying the scores to the patients are shown in Table 14. In addition, the scores for each patient are summed and the results showing distribution of the total score for each patient are shown in Table 15.

[Table 14]

EP 2 492 353 B1

| Group | No. | SUM |
|---|---|---|
| Remarkably effective group | 1 | 12 |
| | 2 | 14 |
| | 3 | 10 |
| | 4 | 0 |
| | 5 | 0 |
| | 6 | 0 |
| | 7 | 12 |
| | 8 | 0 |
| | 9 | 35 |
| | 10 | 39 |
| | 11 | 0 |
| | 12 | 16 |
| | 13 | 31 |
| | 14 | 30 |
| | 15 | 38 |
| | 16 | 15 |
| | 17 | 16 |
| | 18 | 24 |
| | 19 | 3 |
| | 20 | -4 |
| | 21 | 20 |
| | 22 | 0 |
| Ineffective group | 1 | 6 |
| | 2 | 0 |
| | 3 | 0 |
| | 4 | 0 |
| | 5 | -28 |
| | 6 | -8 |
| | 7 | -32 |
| | 8 | 0 |
| | 9 | -17 |
| | 10 | -20 |
| | 11 | -33 |
| | 12 | -30 |
| | 13 | -24 |
| | 14 | 0 |
| | 15 | -11 |
| | 16 | -26 |
| | 17 | -21 |
| | 18 | -28 |
| | 19 | -36 |
| | 20 | -19 |

35

[Table 15]

(people)

| Score | Count | |
|---|---|---|
| | (RE) | (IE) |
| 39 | 1 | 0 |
| 38 | 2 | 0 |
| 37 | 0 | 0 |
| 36 | 0 | 0 |
| 35 | 1 | 0 |
| 34 | 0 | 0 |
| 33 | 0 | 0 |
| 32 | 0 | 0 |
| 31 | 1 | 0 |
| 30 | 1 | 0 |
| 29 | 0 | 0 |
| 28 | 0 | 0 |
| 27 | 0 | 0 |
| 26 | 0 | 0 |
| 25 | 0 | 0 |
| 24 | 1 | 0 |
| 23 | 0 | 0 |
| 22 | 0 | 0 |
| 21 | 0 | 0 |
| 20 | 1 | 0 |
| 19 | 0 | 0 |
| 18 | 0 | 0 |
| 17 | 0 | 0 |
| 16 | 2 | 0 |
| 15 | 1 | 0 |
| 14 | 1 | 0 |
| 13 | 0 | 0 |
| 12 | 2 | 0 |
| 11 | 0 | 0 |
| 10 | 1 | 0 |
| 9 | 0 | 0 |
| 8 | 0 | 0 |
| 7 | 0 | 0 |
| 6 | 0 | 1 |
| 5 | 0 | 0 |
| 4 | 0 | 0 |
| 3 | 1 | 0 |
| 2 | 0 | 0 |
| 1 | 0 | 0 |
| 0 | 5 | 7 |

(cont.)

| | | |
|---|---|---|
| −1 | 0 | 0 |
| −2 | 0 | 0 |
| −3 | 0 | 0 |
| −4 | 1 | 0 |
| −5 | 0 | 0 |
| −6 | 0 | 0 |
| −7 | 0 | 0 |
| −8 | 0 | 1 |
| −9 | 0 | 0 |
| −10 | 0 | 0 |
| −11 | 0 | 1 |
| −12 | 0 | 0 |
| −13 | 0 | 0 |
| −14 | 0 | 0 |
| −15 | 0 | 0 |
| −16 | 0 | 0 |
| −17 | 0 | 1 |
| −18 | 0 | 0 |
| −19 | 0 | 1 |
| −20 | 0 | 1 |
| −21 | 0 | 1 |
| −22 | 0 | 0 |
| −23 | 0 | 0 |
| −24 | 0 | 1 |
| −25 | 0 | 0 |
| −26 | 0 | 1 |
| −27 | 0 | 0 |
| −28 | 0 | 2 |
| −29 | 0 | 0 |
| −30 | 0 | 1 |
| −31 | 0 | 0 |
| −32 | 0 | 1 |
| −33 | 0 | 1 |
| −34 | 0 | 0 |
| −35 | 0 | 0 |
| −36 | 0 | 1 |
| −37 | 0 | 0 |
| −38 | 0 | 0 |
| −39 | 0 | 0 |
| Total | 22 | 22 |

(People)

| | ~−1 | 0 | 1~ | Total |
|---|---|---|---|---|
| (RE) | 1 | 5 | 16 | 22 |
| (IE) | 14 | 7 | 1 | 22 |

(RE): Remarkably Effective
(IE): Ineffective

Selection of target genes were carried out according to the following criteria for each of the variations, i.e., CNV of 1 or 3, where the difference in the numbers of people between the remarkably effective group and the ineffective group was equal to or more than 2.

    (a) Remarkably effective when CNV is 1
    (b) Remarkably effective when CNV is 3
    (c) Ineffective when CNV is 1

(d) Ineffective when CNV is 3

Genes that fall in the above-described criteria are shown in Table 16. In Table 16, circles are marked on the right side for the genes that fall in the above-described criteria.

[Table 16]

| | (RE) | | | (IE) | | | | | (a) Judgable with L(RE) | (b) Judgable with L(IE) | (c) Judgable with G(RE) | (d) Judgable with G(IE) |
|---|---|---|---|---|---|---|---|---|---|---|---|---|
| (RE): Remarkably Effective<br>(IE): Ineffective | | | | | | | | | * Judgment criterion: difference of 2 or more | | | |
| Gene name | L | N | G | L | N | G | L(RE)-L(IE) | G(RE)-G(IE) | | | | |
| CCDC127 | 1 | 6 | 15 | 9 | 12 | 1 | -8 | 14 | | ○ | ○ | |
| **NCAM2** | 11 | 11 | 0 | 0 | 9 | 13 | 11 | -13 | ○ | | | ○ |
| PCDH17 | 7 | 15 | 0 | 1 | 9 | 12 | 6 | -12 | ○ | | | ○ |
| C14orf180 | 0 | 7 | 15 | 10 | 12 | 0 | -10 | 15 | | ○ | ○ | |
| CHODL | 10 | 12 | 0 | 0 | 10 | 12 | 10 | -12 | ○ | | | ○ |
| SIVA1 | 0 | 7 | 15 | 10 | 12 | 0 | -10 | 15 | | ○ | ○ | |
| TNFRSF14 | 1 | 6 | 15 | 12 | 10 | 0 | -11 | 15 | | ○ | ○ | |
| AHNAK2 | 0 | 7 | 15 | 10 | 12 | 0 | -10 | 15 | | ○ | ○ | |
| C14orf79 | 0 | 7 | 15 | 10 | 12 | 0 | -10 | 15 | | ○ | ○ | |
| LOC25845 | 1 | 5 | 16 | 10 | 11 | 1 | -9 | 15 | | ○ | ○ | |
| PLD4 | 0 | 7 | 15 | 10 | 12 | 0 | -10 | 15 | | ○ | ○ | |
| GPR132 | 0 | 7 | 15 | 10 | 12 | 0 | -10 | 15 | | ○ | ○ | |
| LOC389257 | 1 | 6 | 15 | 9 | 12 | 1 | -8 | 14 | | ○ | ○ | |
| BRF1 | 0 | 7 | 15 | 10 | 12 | 0 | -10 | 15 | | ○ | ○ | |
| BTG3 | 10 | 12 | 0 | 0 | 10 | 12 | 10 | -12 | ○ | | | ○ |
| DIAPH3 | 8 | 14 | 0 | 1 | 9 | 12 | 7 | -12 | ○ | | | ○ |
| GBA3 | 6 | 16 | 0 | 0 | 11 | 11 | 6 | -11 | ○ | | | ○ |
| IFRD1 | 8 | 14 | 0 | 0 | 10 | 12 | 8 | -12 | ○ | | | ○ |
| KCNT2 | 7 | 15 | 0 | 0 | 12 | 10 | 7 | -10 | ○ | | | ○ |
| THOC7 | 5 | 17 | 0 | 0 | 14 | 8 | 5 | -8 | ○ | | | ○ |
| **TMEM168** | 8 | 14 | 0 | 0 | 10 | 12 | 8 | -12 | ○ | | | ○ |
| ADSSL1 | 0 | 7 | 15 | 10 | 12 | 0 | -10 | 15 | | ○ | ○ | |

| (RE): Remarkably Effective<br>(IE): Ineffective | | | | | | | | * Judgment criterion: difference of 2 or more | | | | |
| --- | --- | --- | --- | --- | --- | --- | --- | --- | --- | --- | --- | --- |
| Gene name | (RE) | | | (IE) | | | L(RE)-L(IE) | G(RE)-G(IE) | (a) Judgable with L(RE) | (b) Judgable with L(IE) | (c) Judgable with G(RE) | (d) Judgable with G(IE) |
| | L | N | G | L | N | G | | | | | | |
| BST1 | 5 | 17 | 0 | 0 | 10 | 12 | 5 | -12 | ○ | | | ○ |
| C7orf53 | 8 | 14 | 0 | 0 | 10 | 12 | 8 | -12 | ○ | | | ○ |
| CD38 | 5 | 17 | 0 | 0 | 14 | 8 | 5 | -8 | ○ | | | ○ |
| DCUN1D1 | 4 | 18 | 0 | 0 | 21 | 1 | 4 | -1 | ○ | | | |
| FGFBP1 | 5 | 17 | 0 | 0 | 14 | 8 | 5 | -8 | ○ | | | ○ |
| FOXP2 | 8 | 14 | 0 | 0 | 10 | 12 | 8 | -12 | ○ | | | ○ |
| GLRB | 6 | 16 | 0 | 0 | 17 | 5 | 6 | -5 | ○ | | | ○ |
| GTF2B | 4 | 18 | 0 | 0 | 20 | 2 | 4 | -2 | ○ | | | ○ |
| HSP90AB2P | 5 | 17 | 0 | 0 | 14 | 8 | 5 | -8 | ○ | | | ○ |
| MCCC1 | 4 | 18 | 0 | 0 | 21 | 1 | 4 | -1 | ○ | | | |
| MDFIC | 8 | 14 | 0 | 0 | 10 | 12 | 8 | -12 | ○ | | | ○ |
| ODF2L | 4 | 18 | 0 | 0 | 20 | 2 | 4 | -2 | ○ | | | ○ |
| PDGFC | 6 | 16 | 0 | 0 | 17 | 5 | 6 | -5 | ○ | | | ○ |
| SEP15 | 4 | 18 | 0 | 0 | 20 | 2 | 4 | -2 | ○ | | | ○ |
| SH3GLB1 | 4 | 18 | 0 | 0 | 20 | 2 | 4 | -2 | ○ | | | ○ |
| TDRD3 | 8 | 14 | 0 | 1 | 9 | 12 | 7 | -12 | ○ | | | ○ |
| YEATS2 | 4 | 18 | 0 | 0 | 21 | 1 | 4 | -1 | ○ | | | |

EP 2 492 353 B1

As a result, the allergen immunotherapy was found to have a tendency to be effective for NCAM2, PCDH17, CHODL, BTG3, DIAPH3, GBA3, IFRD1, KCNT2, THOC7, TMEM168, BST1, C7orf53, CD38, DCUN1D1, FGFBP1, FOXP2, GLRB, GTF2B, HSP90AB2P, MCCC1, MDFIC, ODF2L, PDGFC, SEP15, SH3GLB1, TDRD3 and YEATS2 when the copy number was 1, while the allergen immunotherapy was found to have a tendency to be effective for CCDC127, C14orf180, SIVA1, TNFRSF14, AHNAK2, C14orf79, LOC25845, PLD4, GPR132, LOC389257, BRF1 and ADSSL1 when the copy number was 3.

On the other hand, the allergen immunotherapy was found to have a tendency to be ineffective for CCDC127, C14orf180,SIVA1, TNFRSF14, AHNAK2, C14orf79, LOC25845, PLD4, GPR132, LOC389257, BRF1 and ADSSL1 when the copy number was 1, while the allergen immunotherapy was found to have a tendency to be ineffective for NCAM2, PCDH17, CHODL, BTG3, DIAPH3, GBA3, IFRD1, KCNT2, THOC7, TMEM168, BST1, C7orf53, CD38, FGFBP1, FOXP2, GLRB, GTF2B, HSP90AB2P, MDFIC, ODF2L, PDGFC, SEP15, SH3GLB1 and TDRD3 when the copy number was 3.

INDUSTRIAL APPLICABILITY

The present invention can detect whether or not allergen immunotherapy is effective in an immediate allergy patient. A method of the present invention can be used for selecting a treatment or a therapeutic drug for an immediate allergy.

**Claims**

1. A method for detecting effectiveness of allergen immunotherapy for an immediate allergy in a subject, the method comprising the steps of: detecting a copy number variation of at least one gene selected from the following gene group in a specimen to be examined collected from the subject; comparing the obtained result from detecting the copy number variation with data of the copy number variation of the same gene from the parent population to correlate with the effectiveness of the allergen immunotherapy:

   <Gene group>
   AGXT2L2, AZGP1, CAV3, CEP72, CUL4A, CXCR7, C12orf60, C16orf48, C19orf34, DNAJB8, FTH1, GEMIN4, GIGYF2, GJC3, HTT, ITCH, MARK2, METT10D, NAV3, NOB1, PAFAH2, PLEKHG4B, PPFIA1, SCARNA11, SDF2L1, SMPD3, SNORA44, SRP14, ST6GALNAC1, SULF2, TMED6, TYRP1, WFDC13, ZNFX1, NCAM2, PCDH17, CHODL, BTG3, DIAPH3, GBA3, IFRD1, KCNT2, THOC7, TMEM168, BST1, C7orf53, CD38, DCUN1D1, FGFBP1, FOXP2, GLRB, GTF2B, HSP90AB2P, MCCC1, MDFIC, ODF2L, PDGFC, SEP15, SH3GLB1, TDRD3, YEATS2, CCDC127, C14orf18, SIVA1, TNFRSF14, AHNAK2, C14orf79, LOC25845, PLD4, GPR132, LOC389257, BRF1 and ADSSL1.

2. The method according to Claim 1, wherein the result from detecting the copy number variation is correlated with effectiveness of the allergen immunotherapy based on any of the following judgment criteria (a) to (d) or a combination thereof:

   (a) the allergen immunotherapy is effective when the copy number of at least one gene selected from the group consisting of NAV3, TYRP1, NCAM2, PCDH17, CHODL, BTG3, DIAPH3, GBA3, IFRD1, KCNT2, THOC7, TMEM168, BST1, C7orf53, CD38, DCUN1D1, FGFBP1, FOXP2, GLRB, GTF2B, HSP90AB2P, MCCC1, MDFIC, ODF2L, PDGFC, SEP15, SH3GLB1, TDRD3 and YEATS2 is smaller than the most frequent copy number of the same gene from the parent population;
   (b) the allergen immunotherapy is effective when the copy number of at least one gene selected from the group consisting of AGXT2L2, AZGP1, CAV3, CEP72, CUL4A, CXCR7, C19orf34, DNAJB8, FTH1, GEMIN4, GIGYF2, GJC3, ITCH, HTT, MARK2, METT10D, PAFAH2, PLEKHG4B, PPFIA1, SCARNA11, SDF2L1, SNORA44, ST6GALNAC1, SULF2, WFDC13, ZNFX1, CCDC127, C14orf180, SIVA1, TNFRSF14, AHNAK2, C14orf79, LOC25845, PLD4, GPR132, LOC389257, BRF1 and ADSSL1 is larger than the most frequent copy number of the same gene from the parent population;
   (c) the allergen immunotherapy is ineffective when the copy number of at least one gene selected from the group consisting of AGXT2L2, CAV3, CEP72, CUL4A, CXCR7, C16orf48, C19orf34, DNAJB8, FTH1, GEMIN4, GIGYF2, GJC3, HTT, ITCH, MARK2, METT10D, NOB1, PAFAH2, PLEKHG4B, SCARNA11, SDF2L1, SMPD3, SNORA44, SRP14, ST6GALNAC1, SULF2, TMED6, WFDC13, ZNFX1, CCDC127, C14orf180, SIVA1, TNFRSF14, AHNAK2, C14orf79, LOC25845, PLD4, GPR132, LOC389257, BRF1 and ADSSL1 is smaller than the most frequent copy number of the same gene from the parent population; and
   (d) the allergen immunotherapy is ineffective when the copy number of at least one gene selected from the group consisting ofC12orf60, NAV3, TYRP1, NCAM2, PCDH17, CHODL, BTG3, DIAPH3, GBA3, IFRD1, KCNT2, THOC7, TMEM168, BST1, C7orf53, CD38, FGFBP1, FOXP2, GLRB, GTF2B, HSP90AB2P, MDFIC, ODF2L, PDGFC, SEP15, SH3GLB1 and TDRD3 is larger than the most frequent copy number of the same gene from the parent population.

3.   The method according to Claim 1, wherein the result from detecting the copy number variation is correlated with effectiveness of the allergen immunotherapy based on any of the following judgement criteria (e) to (h) or a combination thereof:

(e) the allergen immunotherapy is effective when the copy number of at least one gene selected from the group consisting of NAV3, TYRP1, NCAM2, PCDH17, CHODL, BTG3, DIAPH3, GBA3, IFRD1, KCNT2, THOC7, TMEM168, BST1, C7orf53, CD38, DCUN1D1, FGFBP1, FOXP2, GLRB, GTF2B, HSP90AB2P, MCCC1, MDFIC, ODF2L, PDGFC, SEP15, SH3GLB1, TDRD3 and YEATS2 is 1;

(f) the allergen immunotherapy is effective when the copy number of at least one gene selected from the group consisting of AGXT2L2, AZGP1, CAV3, CEP72, CUL4A, CXCR7, C19orf34, DNAJB8, FTH1, GEMIN4, GIGYF2, GJC3, ITCH, HTT, MARK2, METT10D, PAFAH2, PLEKHG4B, PPFIA1, SCARNA11, SDF2L1, SNORA44, ST6GALNAC1, SULF2, WFDC13, ZNFX1, CCDC127, C14orf180, SIVA1, TNFRSF14, AHNAK2, C14orf79, LOC25845, PLD4, GPR132, LOC389257, BRF1 and ADSSL1 is 3;

(g) the allergen immunotherapy is ineffective when the copy number of at least one gene selected from the group consisting of AGXT2L2, CAV3, CEP72, CUL4A, CXCR7, C16orf48, C19orf34, DNAJB8, FTH1, GEMIN4, GIGYF2, GJC3, HTT, ITCH, MARK2, METT10D, NOB1, PAFAH2, PLEKHG4B, SCARNA11, SDF2L1, SMPD3, SNORA44, SRP14, ST6GALNAC1, SULF2, TMED6, WFDC13, ZNFX1, CCDC127, C14orf180, SIVA1, TNFRSF14, AHNAK2, C14orf79, LOC25845, PLD4, GPR132, LOC389257, BRF1 and ADSSL1 is 1; and

(h) the allergen immunotherapy is ineffective when the copy number of at least one gene selected from the group consisting of C12orf60, NAV3, TYRP1, NCAM2, PCDH17, CHODL, BTG3, DIAPH3, GBA3, IFRD1, KCNT2, THOC7, TMEM168, BST1, C7orf53, CD38, FGFBP1, FOXP2, GLRB, GTF2B, HSP90AB2P, MDFIC, ODF2L, PDGFC, SEP15, SH3GLB1 and TDRD3 is 3.

4.   The method according to any one of Claims 1 to 3, wherein the allergen immunotherapy is sublingual allergen immunotherapy.

5.   The method according to any one of Claims 1 to 4, wherein the immediate allergy is at least one selected from the group consisting of pollen allergy, urticaria, food allergy, mite allergy, allergic rhinitis, bronchial asthma and atopic dermatitis.

6.   The method according to any one of Claims 1 to 5, wherein the specimen to be examined is blood or an immune cell.

7.   The method according to any one of Claims 1 to 5, wherein the specimen to be examined is CD4T cell, a dendritic cell or a basophil.

8.   The method according to any one of Claims 1 to 5, wherein the specimen to be examined is mRNA extracted from the cell according to either one of Claims 6 and 7.

9.   The method according to Claim 1, wherein the allergen immunotherapy is sublingual allergen immunotherapy and wherein the immediate allergy is cedar pollen allergy.

10.  The method according to Claim 9, wherein the result from detecting the copy number variation is correlated with effectiveness of the allergen immunotherapy based on any of the following judgment criteria (a) to (d) or a combination thereof:

(a) the allergen immunotherapy is effective when the copy number of at least one gene selected from the group consisting of NAV3, TYRP1, NCAM2, PCDH17, CHODL, BTG3, DIAPH3, GBA3, IFRD1, KCNT2, THOC7, TMEM168, BST1, C7orf53, CD38, DCUN1D1, FGFBP1, FOXP2, GLRB, GTF2B, HSP90AB2P, MCCC1, MDFIC, ODF2L, PDGFC, SEP15, SH3GLB1, TDRD3 and YEATS2 is smaller than the most frequent copy number of the same gene from the parent population;

(b) the allergen immunotherapy is effective when the copy number of at least one gene selected from the group consisting of AGXT2L2, AZGP1, CAV3, CEP72, CUL4A, CXCR7, C19orf34, DNAJB8, FTH1, GEMIN4, GIGYF2, GJC3, ITCH, HTT, MARK2, METT10D, PAFAH2, PLEKHG4B, PPFIA1, SCARNA11, SDF2L1, SNORA44, ST6GALNAC1, SULF2, WFDC13, ZNFX1, CCDC127, C14orf180, SIVA1, TNFRSF14, AHNAK2, C14orf79, LOC25845, PLD4, GPR132, LOC389257, BRF1 and ADSSL1 is larger than the most frequent copy number of the same gene from the parent population;

(c) the allergen immunotherapy is ineffective when the copy number of at least one gene selected from the group consisting of AGXT2L2, CAV3, CEP72, CUL4A, CXCR7, C16orf48, C19orf34, DNAJB8, FTH1, GEMIN4, GIGYF2, GJC3, HTT, ITCH, MARK2, METHOD, NOB1, PAFAH2, PLEKHG4B, SCARNA11, SDF2L1, SMPD3, SNORA44, SRP14, ST6GALNAC1, SULF2, TMED6, WFDC13, ZNFX1, CCDC127, C14orf180, SIVA1, TNFRSF14, AHNAK2, C14orf79, LOC25845, PLD4, GPR132, LOC389257, BRF1 and ADSSL1 is smaller than the most frequent copy number of the same gene from the parent population; and

(d) the allergen immunotherapy is ineffective when the copy number of at least one gene selected from the group consisting of C12orf60, NAV3, TYRP1, NCAM2, PCDH17, CHODL, BTG3, DIAPH3, GBA3, IFRD1, KCNT2, THOC7, TMEM168, BST1, C7orf53, CD38, FGFBP1, FOXP2, GLRB, GTF2B, HSP90AB2P, MDFIC, ODF2L, PDGFC, SEP15, SH3GLB1 and TDRD3 is larger than the most frequent copy number of the same gene from the parent population.

**11.** The method according to Claim 9, wherein the result from detecting the copy number variation is correlated with effectiveness of the allergen based immunotherapy based on any of the following judgement criteria (e) to (h) or a combination thereof:

(e) the allergen immunotherapy is effective when the copy number of at least one gene selected from the group consisting of NAV3, TYRP1, NCAM2, PCDH17, CHODL, BTG3, DIAPH3, GBA3, IFRD1, KCNT2, THOC7, TMEM168, BST1, C7orf53, CD38, DCUN1D1, FGFBP1, FOXP2, GLRB, GTF2B, HSP90AB2P, MCCC1, MDFIC, ODF2L, PDGFC, SEP15, SH3GLB1, TDRD3 and YEATS2 is 1;

(f) the allergen immunotherapy is effective when the copy number of at least one gene selected from the group consisting of AGXT2L2, AZGP1, CAV3, CEP72, CUL4A, CXCR7, C19orf34, DNAJB8, FTH1, GEMIN4, GIGYF2, GJC3, ITCH, HTT, MARK2, METT10D, PAFAH2, PLEKHG4B, PPFIA1, SCARNA11, SDF2L1, SNORA44, ST6GALNAC1, SULF2, WFDC13, ZNFX1, CCDC127, C14orf180, SIVA1, TNFRSF14, AHNAK2, C14orf79, LOC25845, PLD4, GPR132, LOC389257, BRF1 and ADSSL1 is 3;

(g) the allergen immunotherapy is ineffective when the copy number of at least one gene selected from the group consisting of AGXT2L2, CAV3, CEP72, CUL4A, CXCR7, C16orf48, C19orf34, DNAJB8, FTH1, GEMIN4, GIGYF2, GJC3, HTT, ITCH, MARK2, METT10D, NOB1, PAFAH2, PLEKHG4B, SCARNA11, SDF2L1, SMPD3, SNORA44, SRP14, ST6GALNAC1, SULF2, TMED6, WFDC13, ZNFX1, CCDC127, C14orf180, SIVA1, TNFRSF14, AHNAK2, C14orf79, LOC25845, PLD4, GPR132, LOC389257, BRF1 and ADSSL1 is 1; and

(h) the allergen immunotherapy is ineffective when the copy number of at least one gene selected from the group consisting of C12orf60, NAV3, TYRP1, NCAM2, PCDH17, CHODL, BTG3, DIAPH3, GBA3, IFRD1, KCNT2, THOC7, TMEM168, BST1, C7orf53, CD38, FGFBP1, FOXP2, GLRB, GTF2B, HSP90AB2P, MDFIC, ODF2L, PDGFC, SEP15, SH3GLB1 and TDRD3 is 3.

**12.** The method according to any one of Claims 9 to 11, wherein the specimen to be examined is blood or an immune cell.

**13.** The method according to any one of Claims 9 to 11, wherein the specimen to be examined is CD4T cell, a dendritic cell or a basophil.

**14.** The method according to any one of Claims 9 to 11, wherein the specimen to be examined is mRNA extracted from the cell according to either one of Claims 12 and 13.

**Patentansprüche**

**1.** Verfahren zur Ermittlung der Wirksamkeit einer Hyposensibilisierungstherapie einer Allergie des Soforttyps bei einem Subjekt, wobei das Verfahren die Schritte umfasst:

Ermittlung einer Veränderung der Vervielfältigungszahl von mindestens einem Gen, ausgewählt von der nachfolgenden Gengruppe, in einer Probe, die von dem Subjekt zur Untersuchung entnommen worden ist; Vergleich des erhaltenen Ergebnis aus der Ermittlung der Veränderung der Vervielfältigungszahl mit Daten der Veränderung der Vervielfältigungszahl des gleichen Gens der Stammpopulation, um dies mit der Wirksamkeit der Hyposensibilisierungstherapie zu korrelieren:

<Gengruppe>

AGXT2L2, AZGP1, CAV3, CEP72, CUL4A, CXCR7, C12orf60, C16orf48, C19orf34, DNAJB8, FTH1, GEMIN4, GIGYF2, GJC3, HTT, ITCH, MARK2, METT10D, NAV3, NOB1, PAFAH2, PLEKHG4B, PPFIAI, SCARNA11, SDF2L1, SMPD3, SNORA44, SRP14, ST6GALNAC1, SULF2, TMED6, TYRP1, WFDC13, ZNFX1, NCAM2, PCDH17, CHODL, BTG3, DIAPH3, GBA3, IFRD1, KCNT2, THOC7, TMEM168, BST1, C7orf53, CD38, DCUN1DI, FGFBP1, FOXP2, GLRB, GTF2B, HSP90AB2P, MCCC1, MDFIC, ODF2L, PDGFC, SEP15, SH3GLB1, TDRD3, YEATS2, CCDC127, C14orf180, SIVA1, TNFRSF14, AHNAK2, C14orf79, LOC25845, PLD4, GPR132, LOC389257, BRF1 und ADSSL1.

2. Verfahren gemäß Anspruch 1, wobei das Ergebnis der Ermittlung der Veränderung der Vervielfältigungszahl mit der Wirksamkeit der Hyposensibilisierungstherapie basierend auf einem der nachfolgenden Beurteilungskriterien (a) bis (d), oder einer Kombination davon, korreliert wird:

(a) die Hyposensibilisierungstherapie ist wirksam, wenn die Vervielfältigungszahl von mindestens einem Gen, ausgewählt aus der Gruppe, bestehend aus NAV3, TYRP1, NCAM2, PCDH17, CHODL, BTG3, DIAPH3, GBA3, IFRD1, KCNT2, THOC7, TMEM168, BST1, C7orf53, CD38, DCUN1D1, FGFBP1, FOXP2, GLRB, GTF2B, HSP90AB2P, MCCC1, MDFIC, ODF2L, PDGFC, SEP15, SH3GLB1, TDRD3 und YEATS2 kleiner ist als die häufigste Vervielfältigungszahl des gleichen Gens aus der Stammpopulation;
(b) die Hyposensibilisierungstherapie ist wirksam, wenn die Vervielfältigungszahl von mindestens einem Gen, ausgewählt aus der Gruppe, bestehend aus AGXT2L2, AZGP1, CAV3, CEP72, CUL4A, CXCR7, C19orf34, DNAJB8, FTH1, GEMIN4, GIGYF2, GJC3, ITCH, HTT, MARK2, METT10D, PAFAH2, PLEKHG4B, PPFIA1, SCARNA11, SDF2L1, SNORA44, ST6GALNAC1, SULF2, WFDC13, ZNFX1, CCDC127, C14orf180, SIVA1, TNFRSF14, AHNAK2, C14orf79, LOC25845, PLD4, GPR132, LOC389257, BRF1 und ADSSL1 größer ist als die häufigste Vervielfältigungszahl des gleichen Gens aus der Stammpopulation;
(c) die Hyposensibilisierungstherapie ist unwirksam, wenn die Vervielfältigungszahl von mindestens einem Gen, ausgewählt aus der Gruppe, bestehend aus AGXT2L2, CAV3, CEP72, CUL4A, CXCR7, C16orf48, C19orf34, DNAJB8, FTH1, GEMIN4, GIGYF2, GJC3, HTT, ITCH, MARK2, METT10D, NOB1, PAFAH2, PLEKHG4B, SCARNA11, SDF2L1, SMPD3, SNORA44, SRP14, ST6GALNAC1, SULF2, TMED6, WFDC13, ZNFX1, CCDC127, C14orfl80, SIVA1, TNFRSF14, AHNAK2, Cl4orf79, LOC25845, PLD4, GPR132, LOC389257, BRF1 und ADSSL1 kleiner ist als die häufigste Vervielfältigungszahl des gleichen Gens aus der Stammpopulation; und
(d) die Hyposensibilisierungstherapie ist unwirksam, wenn die Vervielfältigungszahl von mindestens einem Gen, ausgewählt aus der Gruppe, bestehend aus C12orf60, NAV3, TYRP1, NCAM2, PCDH17, CHODL, BTG3, DIAPH3, GBA3, IFRD1, KCNT2, THOC7, TMEM168, BST1, C7orf53, CD38, FGFBP1, FOXP2, GLRB, GTF2B, HSP90AB2P, MDFIC, ODF2L, PDGFC, SEP15, SH3GLB1 und TDRD3 größer ist als die häufigste Vervielfältigungszahl des gleichen Gens aus der Stammpopulation.

3. Verfahren gemäß Anspruch 1, wobei das Ergebnis der Ermittlung der Veränderung der Vervielfältigungszahl mit der Wirksamkeit der Hyposensibilisierungstherapie basierend auf einer der folgenden Beurteilungskriterien (e) bis (h), oder einer Kombination davon, korreliert wird:

(e) die Hyposensibilisierungstherapie ist wirksam, wenn die Vervielfältigungszahl von mindestens einem Gen, ausgewählt aus der Gruppe, bestehend aus NAV3, TYRP1, NCAM2, PCDH17, CHODL, BTG3, DIAPH3, GBA3, IFRD1, KCNT2, THOC7, TMEM168, BST1, C7orf53, CD38, DCUN1D1, FGFBP1, FOXP2, GLRB, GTF2B, HSP90AB2P, MCCC1, MDFIC, ODF2L, PDGFC, SEP15, SH3GLB1, TDRD3 und YEATS2 1 ist;
(f) die Hyposensibilisierungstherapie ist wirksam, wenn die Vervielfältigungszahl von mindestens einem Gen, ausgewählt aus der Gruppe, bestehend aus AGXT2L2, AZGP1, CAV3, CEP72, CUL4A, CXCR7, C19orf34, DNAJB8, FTH1, GEMIN4, GIGYF2, GJC3, ITCH, HTT, MARK2, METT10D, PAFAH2, PLEKHG4B, PPFIA1, SCARNA11, SDF2L1, SNORA44, ST6GALNAC1, SULF2, WFDC13, ZNFX1, CCDC127, C14orf180, SIVA1, TNFRSF14, AHNAK2, C14orf79, LOC25845, PLD4, GPR132, LOC389257, BRF1 und ADSSL1 3 ist;
(g) die Hyposensibilisierungstherapie ist unwirksam, wenn die Vervielfältigungszahl von mindestens einem Gen, ausgewählt aus der Gruppe, bestehend aus AGXT2L2, CAV3, CEP72, CUL4A, CXCR7, C16orf48, C19orf34, DNAJB8, FTH1, GEMIN4, GIGYF2, GJC3, HTT, ITCH, MARK2, METT10D, NOB1, PAFAH2, PLEKHG4B, SCARNA11, SDF2L1, SMPD3, SNORA44, SRP14, ST6GALNAC1, SULF2, TMED6, WFDC13, ZNFX1, CCDC127, C14orf180, SIVA1, TNFRSF14, AHNAK2, C14orf79, LOC25845, PLD4, GPR132, LOC389257, BRF1 und ADSSL1 1 ist; und
(h) die Hyposensibilisierungstherapie ist unwirksam, wenn die Vervielfältigungszahl von mindestens einem Gen, ausgewählt aus der Gruppe, bestehend aus C12orf60, NAV3, TYRP1, NCAM2, PCDH17, CHODL, BTG3, DIAPH3, GBA3, IFRD1, KCNT2, THOC7, TMEM168, BST1, C7orf53, CD38, FGFBP1, FOXP2, GLRB, GTF2B, HSP90AB2P, MDFIC, ODF2L, PDGFC, SEP15, SH3GLB1 und TDRD3 3 ist.

4. Verfahren gemäß einem der Ansprüche 1 bis 3, wobei die Hyposensibilisierungstherapie eine sublinguale Hyposensibilisierungstherapie ist.

5. Verfahren gemäß einem der Ansprüche 1 bis 4, wobei die Allergie des Soforttyps mindestens eine, ausgewählt aus der Gruppe, bestehend aus Pollenallergie, Nesselsucht, Lebensmittelallergie, Milbenallergie, allergischer Rhinitis, bronchialem Asthma und atopischer Dermatitis ist.

6. Verfahren gemäß einem der Ansprüche 1 bis 5, wobei die zu untersuchende Probe Blut oder eine Immunzelle ist.

7. Verfahren gemäß einem der Ansprüche 1 bis 5, wobei die zu untersuchende Probe eine CD4T-Zelle, eine dendritische Zelle oder ein basophiler Granulozyt ist.

8. Verfahren gemäß einem der Ansprüche 1 bis 5, wobei die zu untersuchende Probe aus einer Zelle gemäß einer der Ansprüche 6 und 7 extrahierte mRNA ist.

9. Verfahren gemäß Anspruch 1, wobei die Hyposensibilisierungstherapie eine sublinguale Hyposensibilisierungstherapie ist und wobei die Allergie des Soforttyps Zedernpollenallergie ist.

10. Verfahren gemäß Anspruch 9, wobei das Ergebnis der Ermittlung der Veränderung der Vervielfältigungszahl mit der Wirksamkeit der Hyposensibilisierungstherapie basierend auf einer der nachfolgenden Beurteilungskriterien (a) bis (d), oder einer Kombination davon, korreliert wird:

(a) die Hyposensibilisierungstherapie ist wirksam, wenn die Vervielfältigungszahl von mindestens einem Gen, ausgewählt aus der Gruppe, bestehend aus NAV3, TYRP1, NCAM2, PCDH17, CHODL, BTG3, DIAPH3, GBA3, IFRD1, KCNT2, THOC7, TMEM168, BST1, C7orf53, CD38, DCUN1D1, FGFBP1, FOXP2, GLRB, GTF2B, HSP90AB2P, MCCC1, MDFIC, ODF2L, PDGFC, SEP15, SH3GLB1, TDRD3 und YEATS2 kleiner ist als die häufigste Vervielfältigungszahl des gleichen Gens aus der Stammpopulation;
(b) die Hyposensibilisierungstherapie ist wirksam, wenn die Vervielfältigungszahl von mindestens einem Gen, ausgewählt aus der Gruppe, bestehend aus AGXT2L2, AZGP1, CAV3, CEP72, CUL4A, CXCR7, C19orf34, DNAJB8, FTH1, GEMIN4, GIGYF2, GJC3, ITCH, HTT, MARK2, METT10D, PAFAH2, PLEKHG4B, PPFIA1, SCARNA11, SDF2L1, SNORA44, ST6GALNAC1, SULF2, WFDC13, ZNFX1, CCDC127, C14orf180, SIVA1, TNFRSF14, AHNAK2, C14orf79, LOC25845, PLD4, GPR132, LOC389257, BRF1 und ADSSL1 größer ist als die häufigste Vervielfältigungszahl des gleichen Gens aus der Stammpopulation;
(c) die Hyposensibilisierungstherapie ist unwirksam, wenn die Vervielfältigungszahl von mindestens einem Gen, ausgewählt aus der Gruppe, bestehend aus AGXT2L2, CAV3, CEP72, CUL4A, CXCR7, C16orf48, C19orf34, DNAJB8, FTH1, GEMIN4, GIGYF2, GJC3, HTT, ITCH, MARK2, METT10D, NOB1, PAFAH2, PLEKHG4B, SCARNA11, SDF2L1, SMPD3, SNORA44, SRP14, ST6GALNAC1, SULF2, TMED6, WFDC13, ZNFX1, CCDC127, C14orfl80, SIVA1, TNFRSF14, AHNAK2, Cl4orf79, LOC25845, PLD4, GPR132, LOC389257, BRF1 und ADSSL1 kleiner ist als die häufigste Vervielfältigungszahl des gleichen Gens aus der Stammpopulation; und
(d) die Hyposensibilisierungstherapie ist unwirksam, wenn die Vervielfältigungszahl von mindestens einem Gen, ausgewählt aus der Gruppe, bestehend aus C12orf60, NAV3, TYRP1, NCAM2, PCDH17, CHODL, BTG3, DIAPH3, GBA3, IFRD1, KCNT2, THOC7, TMEM168, BST1, C7orf53, CD38, FGFBP1, FOXP2, GLRB, GTF2B, HSP90AB2P, MDFIC, ODF2L, PDGFC, SEP15, SH3GLB1 und TDRD3 größer ist als die häufigste Vervielfältigungszahl des gleichen Gens aus der Stammpopulation.

11. Verfahren gemäß Anspruch 9, wobei das Ergebnis der Ermittlung der Veränderung der Vervielfältigungszahl mit der Wirksamkeit der Hyposensibilisierungstherapie basierend auf einer der nachfolgenden Beurteilungskriterien (e) bis (h), oder einer Kombination davon, korreliert wird:

(e) die Hyposensibilisierungstherapie ist wirksam, wenn die Vervielfältigungszahl von mindestens einem Gen, ausgewählt aus der Gruppe, bestehend aus NAV3, TYRP1, NCAM2, PCDH17, CHODL, BTG3, DIAPH3, GBA3, IFRD1, KCNT2, THOC7, TMEM168, BST1, C7orf53, CD38, DCUN1D1, FGFBP1, FOXP2, GLRB, GTF2B, HSP90AB2P, MCCC1, MDFIC, ODF2L, PDGFC, SEP15, SH3GLB1, TDRD3 und YEATS2 1 ist;
(f) die Hyposensibilisierungstherapie ist wirksam, wenn die Vervielfältigungszahl von mindestens einem Gen, ausgewählt aus der Gruppe, bestehend aus AGXT2L2, AZGP1, CAV3, CEP72, CUL4A, CXCR7, C19orf34, DNAJB8, FTH1, GEMIN4, GIGYF2, GJC3, ITCH, HTT, MARK2, METT10D, PAFAH2, PLEKHG4B, PPFIA1, SCARNA11, SDF2L1, SNORA44, ST6GALNAC1, SULF2, WFDC13, ZNFX1, CCDC127, C14orf180, SIVA1, TNFRSF14, AHNAK2, C14orf79, LOC25845, PLD4, GPR132, LOC389257, BRF1 und ADSSL1 3 ist;

(g) die Hyposensibilisierungstherapie ist unwirksam, wenn die Vervielfältigungszahl von mindestens einem Gen, ausgewählt aus der Gruppe, bestehend aus AGXT2L2, CAV3, CEP72, CUL4A, CXCR7, C16orf48, C19orf34, DNAJB8, FTH1, GEMIN4, GIGYF2, GJC3, HTT, ITCH, MARK2, METT10D, NOB1, PAFAH2, PLEKHG4B, SCARNA11, SDF2L1, SMPD3, SNORA44, SRP14, ST6GALNAC1, SULF2, TMED6, WFDC13, ZNFX1, CCDC127, C14orfl80, SIVA1, TNFRSF14, AHNAK2, Cl4orf79, LOC25845, PLD4, GPR132, LOC389257, BRF1 und ADSSL1 1 ist; und

(h) die Hyposensibilisierungstherapie ist unwirksam, wenn die Vervielfältigungszahl von mindestens einem Gen, ausgewählt aus der Gruppe, bestehend aus C12orf60, NAV3, TYRP1, NCAM2, PCDH17, CHODL, BTG3, DIAPH3, GBA3, IFRD1, KCNT2, THOC7, TMEM168, BST1, C7orf53, CD38, FGFBP1, FOXP2, GLRB, GTF2B, HSP90AB2P, MDFIC, ODF2L, PDGFC, SEP15, SH3GLB1 und TDRD3 3 ist.

12. Verfahren gemäß einem der Ansprüche 9 bis 11, wobei die zu untersuchende Probe Blut oder eine Immunzelle ist.

13. Verfahren gemäß einem der Ansprüche 9 bis 11, wobei die zu untersuchende Probe eine CD4T-Zelle, eine dendritische Zelle oder ein basophiler Granulozyt ist.

14. Verfahren gemäß einem der Ansprüche 9 bis 11, wobei die zu untersuchende Probe aus der Zelle gemäß einer der Ansprüche 12 und 13 extrahierte mRNA ist.


## Revendications

1. Procédé de détection de l'efficacité d'une immunothérapie par allergène pour une allergie immédiate chez un sujet, lequel procédé comporte les étapes suivantes :

- détecter une variation du nombre de copies d'au moins un gène, choisi dans l'ensemble de gènes indiqué ci-dessous, dans un échantillon pour examen prélevé sur le sujet ;
- comparer le résultat issu de cette détection d'une variation du nombre de copies avec les données de variation du nombre de copies du même gène au sein de la parentèle, pour le corréler avec l'efficacité de l'immunothérapie par allergène ;
Ensemble de gènes :

AGXT2L2, AZGP1, CAV3, CEP72, CUL4A, CXCR7, C12orf60, C16orf48, C19orf34, DNAJB8, FTH1, GEMIN4, GIGYF2, GJC3, HTT, ITCH, MARK2, METT10D, NAV3, NOB1, PAFAH2, PLEKHG4B, PPFIA1, SCARNA11, SDF2L1, SMPD3, SNORA44, SRP14, ST6GALNAC1, SULF2, TMED6, TYRP1, WFDC13, ZNFX1, NCAM2, PCDH17, CHODL, BTG3, DIAPH3, GBA3, IFRD1, KCNT2, THOC7, TMEM168, BST1, C7orf53, CD38, DCUN1D1, FGFBP1, FOXP2, GLRB, GTF2B, HSP90AB2P, MCCC1, MDFIC, ODF2L, PDGFC, SEP15, SH3GLB1, TDRD3, YEATS2, CCDC127, C14orfl80, SIVA1, TNFRSF14, AHNAK2, C14orf179, LOC25845, PLD4, GPR132, LOC389257, BRF1 et ADSSL1.

2. Procédé conforme à la revendication 1, dans lequel on corrèle le résultat issu de la détection d'une variation du nombre de copies avec l'efficacité de l'immunothérapie par allergène en se basant sur l'un des critères de jugement (a) à (d) suivants ou sur une combinaison de ces critères :

a) l'immunothérapie par allergène est efficace quand le nombre de copies d'au moins un gène choisi dans l'ensemble formé par les suivants :

NAV3, TYRP1, NCAM2, PCDH17, CHODL, BTG3, DIAPH3, GBA3, IFRD1, KCNT2, THOC7, TMEM168, BST1, C7orf53, CD38, DCUN1D1, FGFBP1, FOXP2, GLRB, GTF2B, HSP90AB2P, MCCC1, MDFIC, ODF2L, PDGFC, SEP15, SH3GLB1, TDRD3 et YEATS2,
est plus petit que la valeur la plus fréquente du nombre de copies du même gène au sein de la parentèle ;

b) l'immunothérapie par allergène est efficace quand le nombre de copies d'au moins un gène choisi dans l'ensemble formé par les suivants :

AGXT2L2, AZGP1, CAV3, CEP72, CUL4A, CXCR7, C19orf34, DNAJB8, FTH1, GEMIN4, GIGYF2, GJC3, ITCH, HTT, MARK2, METT10D, PAFAH2, PLEKHG4B, PPFIA1, SCARNA11, SDF2L1, SNORA44, ST6GALNAC1, SULF2, WFDC13, ZNFX1, CCDC127, C14orf180, SIVA1, TNFRSF14, AHNAK2, C14orf179, LOC25845, PLD4, GPR132, LOC389257, BRF1 et ADSSL1, est plus grand que la valeur la plus fréquente du nombre de copies du même gène au sein de la parentèle ;

c) l'immunothérapie par allergène est inefficace quand le nombre de copies d'au moins un gène choisi dans l'ensemble formé par les suivants :

AGXT2L2, CAV3, CEP72, CUL4A, CXCR7, C16orf48, C19orf34, DNAJB8, FTH1, GEMIN4, GIGYF2, GJC3, ITCH, HTT, MARK2, METT10D, NOB1, PAFAH2, PLEKHG4B, SCARNA11, SDF2L1, SMPD3, SNORA44, SRP14, ST6GALNAC1, SULF2, TMED6, WFDC13, ZNFX1, CCDC127, C14orf180, SIVA1, TNFRSF14, AHNAK2, C14orf179, LOC25845, PLD4, GPR132, LOC389257, BRF1 et ADSSL1, est plus petit que la valeur la plus fréquente du nombre de copies du même gène au sein de la parentèle ;

d) l'immunothérapie par allergène est inefficace quand le nombre de copies d'au moins un gène choisi dans l'ensemble formé par les suivants :

C12orf60, NAV3, TYRP1, NCAM2, PCDH17, CHODL, BTG3, DIAPH3, GBA3, IFRD1, KCNT2, THOC7, TMEM168, BST1, C7orf53, CD38, FGFBP1, FOXP2, GLRB, GTF2B, HSP90AB2P, MDFIC, ODF2L, PDGFC, SEP15, SH3GLB1 et TDRD3, est plus grand que la valeur la plus fréquente du nombre de copies du même gène au sein de la parentèle.

3. Procédé conforme à la revendication 1, dans lequel on corrèle le résultat issu de la détection d'une variation du nombre de copies avec l'efficacité de l'immunothérapie par allergène en se basant sur l'un des critères de jugement (e) à (h) suivants ou sur une combinaison de ces critères :

e) l'immunothérapie par allergène est efficace quand le nombre de copies d'au moins un gène choisi dans l'ensemble formé par les suivants :

NAV3, TYRP1, NCAM2, PCDH17, CHODL, BTG3, DIAPH3, GBA3, IFRD1, KCNT2, THOC7, TMEM168, BST1, C7orf53, CD38, DCUN1D1, FGFBP1, FOXP2, GLRB, GTF2B, HSP90AB2P, MCCC1, MDFIC, ODF2L, PDGFC, SEP15, SH3GLB1, TDRD3 et YEATS2, vaut 1 ;

f) l'immunothérapie par allergène est efficace quand le nombre de copies d'au moins un gène choisi dans l'ensemble formé par les suivants :

AGXT2L2, AZGP1, CAV3, CEP72, CUL4A, CXCR7, C19orf34, DNAJB8, FTH1, GEMIN4, GIGYF2, GJC3, ITCH, HTT, MARK2, METT10D, PAFAH2, PLEKHG4B, PPFIA1, SCARNA11, SDF2L1, SNORA44, ST6GALNAC1, SULF2, WFDC13, ZNFX1, CCDC127, C14orf180, SIVA1, TNFRSF14, AHNAK2, C14orfl79, LOC25845, PLD4, GPR132, LOC389257, BRF1 et ADSSL1, vaut 3 ;

g) l'immunothérapie par allergène est inefficace quand le nombre de copies d'au moins un gène choisi dans l'ensemble formé par les suivants :

AGXT2L2, CAV3, CEP72, CUL4A, CXCR7, C16orf48, C19orf34, DNAJB8, FTH1, GEMIN4, GIGYF2, GJC3, ITCH, HTT, MARK2, METT10D, NOB1, PAFAH2, PLEKHG4B, SCARNA11, SDF2L1, SMPD3, SNORA44, SRP14, ST6GALNAC1, SULF2, TMED6, WFDC13, ZNFX1, CCDC127, C14orf180, SIVA1, TNFRSF14, AHNAK2, C14orf179, LOC25845, PLD4, GPR132, LOC389257, BRF1 et ADSSL1, vaut 1 ;

h) l'immunothérapie par allergène est inefficace quand le nombre de copies d'au moins un gène choisi dans l'ensemble formé par les suivants :

C12orf60, NAV3, TYRP1, NCAM2, PCDH17, CHODL, BTG3, DIAPH3, GBA3, IFRD1, KCNT2, THOC7, TMEM168, BST1, C7orf53, CD38, FGFBP1, FOXP2, GLRB, GTF2B, HSP90AB2P, MDFIC, ODF2L, PDGFC, SEP15, SH3GLB1 et TDRD3,

vaut 3.

4. Procédé conforme à l'une des revendications 1 à 3, dans lequel l'immunothérapie par allergène est l'immunothérapie par allergène par voie sublinguale.

5. Procédé conforme à l'une des revendications 1 à 4, dans lequel l'allergie immédiate est au moins une allergie choisie dans l'ensemble constitué par les suivantes : allergie au pollen, urticaire, allergie alimentaire, allergie aux acariens, rhinite allergique, asthme bronchique et dermatite atopique.

6. Procédé conforme à l'une des revendications 1 à 5, dans lequel l'échantillon à examiner est du sang ou des cellules immunitaires.

7. Procédé conforme à l'une des revendications 1 à 5, dans lequel l'échantillon à examiner est des lymphocytes T CD4+, des cellules dendritiques ou des cellules basophiles.

8. Procédé conforme à l'une des revendications 1 à 5, dans lequel l'échantillon à examiner est de l'ARNm extrait de cellules mentionnées dans l'une des revendications 6 et 7.

9. Procédé conforme à la revendication 1, dans lequel l'immunothérapie par allergène est l'immunothérapie par allergène par voie sublinguale et dans lequel l'allergie immédiate est l'allergie au pollen de cèdre.

10. Procédé conforme à la revendication 9, dans lequel on corrèle le résultat issu de la détection d'une variation du nombre de copies avec l'efficacité de l'immunothérapie par allergène en se basant sur l'un des critères de jugement (a) à (d) suivants ou sur une combinaison de ces critères :

a) l'immunothérapie par allergène est efficace quand le nombre de copies d'au moins un gène choisi dans l'ensemble formé par les suivants :

NAV3, TYRP1, NCAM2, PCDH17, CHODL, BTG3, DIAPH3, GBA3, IFRD1, KCNT2, THOC7, TMEM168, BST1, C7orf53, CD38, DCUN1D1, FGFBP1, FOXP2, GLRB, GTF2B, HSP90AB2P, MCCC1, MDFIC, ODF2L, PDGFC, SEP15, SH3GLB1, TDRD3 et YEATS2,
est plus petit que la valeur la plus fréquente du nombre de copies du même gène au sein de la parentèle ;

b) l'immunothérapie par allergène est efficace quand le nombre de copies d'au moins un gène choisi dans l'ensemble formé par les suivants :

AGXT2L2, AZGP1, CAV3, CEP72, CUL4A, CXCR7, C19orf34, DNAJB8, FTH1, GEMIN4, GIGYF2, GJC3, ITCH, HTT, MARK2, METT10D, PAFAH2, PLEKHG4B, PPFIA1, SCARNA11, SDF2L1, SNORA44, ST6GALNAC1, SULF2, WFDC13, ZNFX1, CCDC127, C14orf180, SIVA1, TNFRSF14, AHNAK2, C14orf179, LOC25845, PLD4, GPR132, LOC389257, BRF1 et ADSSL1,
est plus grand que la valeur la plus fréquente du nombre de copies du même gène au sein de la parentèle ;

c) l'immunothérapie par allergène est inefficace quand le nombre de copies d'au moins un gène choisi dans l'ensemble formé par les suivants :

AGXT2L2, CAV3, CEP72, CUL4A, CXCR7, C16orf48, C19orf34, DNAJB8, FTH1, GEMIN4, GIGYF2, GJC3, ITCH, HTT, MARK2, METT10D, NOB1, PAFAH2, PLEKHG4B, SCARNA11, SDF2L1, SMPD3, SNORA44, SRP14, ST6GALNAC1, SULF2, TMED6, WFDC13, ZNFX1, CCDC127, C14orf180, SIVA1, TNFRSF14, AHNAK2, C14orf179, LOC25845, PLD4, GPR132, LOC389257, BRF1 et ADSSL1,
est plus petit que la valeur la plus fréquente du nombre de copies du même gène au sein de la parentèle ;

d) l'immunothérapie par allergène est inefficace quand le nombre de copies d'au moins un gène choisi dans l'ensemble formé par les suivants :

C12orf60, NAV3, TYRP1, NCAM2, PCDH17, CHODL, BTG3, DIAPH3, GBA3, IFRD1, KCNT2, THOC7, TMEM168, BST1, C7orf53, CD38, FGFBP1, FOXP2, GLRB, GTF2B, HSP90AB2P, MDFIC, ODF2L, PDGFC, SEP15, SH3GLB1 et TDRD3,
est plus grand que la valeur la plus fréquente du nombre de copies du même gène au sein de la parentèle.

**11.** Procédé conforme à la revendication 9, dans lequel on corrèle le résultat issu de la détection d'une variation du nombre de copies avec l'efficacité de l'immunothérapie par allergène en se basant sur l'un des critères de jugement (e) à (h) suivants ou sur une combinaison de ces critères :

e) l'immunothérapie par allergène est efficace quand le nombre de copies d'au moins un gène choisi dans l'ensemble formé par les suivants :

NAV3, TYRP1, NCAM2, PCDH17, CHODL, BTG3, DIAPH3, GBA3, IFRD1, KCNT2, THOC7, TMEM168, BST1, C7orf53, CD38, DCUN1D1, FGFBP1, FOXP2, GLRB, GTF2B, HSP90AB2P, MCCC1, MDFIC, ODF2L, PDGFC, SEP15, SH3GLB1, TDRD3 et YEATS2,
vaut 1 ;

f) l'immunothérapie par allergène est efficace quand le nombre de copies d'au moins un gène choisi dans l'ensemble formé par les suivants :

AGXT2L2, AZGP1, CAV3, CEP72, CUL4A, CXCR7, C19orf34, DNAJB8, FTH1, GEMIN4, GIGYF2, GJC3, ITCH, HTT, MARK2, METT10D, PAFAH2, PLEKHG4B, PPFIA1, SCARNA11, SDF2L1, SNORA44, ST6GALNAC1, SULF2, WFDC13, ZNFX1, CCDC127, Cl4orf180, SIVA1, TNFRSF14, AHNAK2, C14orf179, LOC25845, PLD4, GPR132, LOC389257, BRF1 et ADSSL1,
vaut 3 ;

g) l'immunothérapie par allergène est inefficace quand le nombre de copies d'au moins un gène choisi dans l'ensemble formé par les suivants :

AGXT2L2, CAV3, CEP72, CUL4A, CXCR7, C16orf48, C19orf34, DNAJB8, FTH1, GEMIN4, GIGYF2, GJC3, ITCH, HTT, MARK2, METT10D, NOB1, PAFAH2, PLEKHG4B, SCARNA11, SDF2L1, SMPD3, SNORA44, SRP14, ST6GALNAC1, SULF2, TMED6, WFDC13, ZNFX1, CCDC127, C14orf180, SIVA1, TNFRSF14, AHNAK2, C14orf179, LOC25845, PLD4, GPR132, LOC389257, BRF1 et ADSSL1,
vaut 1 ;

h) l'immunothérapie par allergène est inefficace quand le nombre de copies d'au moins un gène choisi dans l'ensemble formé par les suivants :

C12orf60, NAV3, TYRP1, NCAM2, PCDH17, CHODL, BTG3, DIAPH3, GBA3, IFRD1, KCNT2, THOC7, TMEM168, BST1, C7orf53, CD38, FGFBP1, FOXP2, GLRB, GTF2B, HSP90AB2P, MDFIC, ODF2L, PDGFC, SEP15, SH3GLB1 et TDRD3,
vaut 3.

**12.** Procédé conforme à l'une des revendications 9 à 11, dans lequel l'échantillon à examiner est du sang ou des cellules immunitaires.

**13.** Procédé conforme à l'une des revendications 9 à 11, dans lequel l'échantillon à examiner est des lymphocytes T CD4+, des cellules dendritiques ou des cellules basophiles.

**14.** Procédé conforme à l'une des revendications 9 à 11, dans lequel l'échantillon à examiner est de l'ARNm extrait de cellules mentionnées dans l'une des revendications 12 et 13.

Figure 1

Study Plan for serological and genetic search for sublingual hyposensitization therapy (Treatment duration and timetable for blood drawing)

Figure 2

Change in clinical condition upon sublingual hyposensitization therapy and judgment of therapeutic efficacy thereof

Figure 3

Figure 4

Figure 5

Figure 6

Figure 7

Figure 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **REBIEN W. et al.** *Eur J Pediatr.,* July 1982, vol. 138 (4), 341-4
- **PASSALACQUA G. et al.** *Canonica GW. BioDrugs.,* 2001, vol. 15 (8), 509-19
- **GUEZ S et al.** *Allergy,* April 2000, vol. 55 (4), 369-75
- **SAMBROOK ; FRITSCH ; MANIATIS.** Molecular Cloning: A Laboratory Manual. Cold Spring Harbor Laboratory Press, 1989